# EUROPEAN PATENT SPECIFICATION

(11) **EP 3 146 072 B1**
(45) Date of publication and mention of the grant of the patent: **10.04.2019**
(21) Application number: 15728774.9
(22) Date of filing: 22.05.2015
(51) Int. Cl.: C12Q 1/6883

(54) **CIRCULATING MIRNAS AS BIOMARKERS OF THERAPY EFFECTIVENESS IN RHEUMATOID ARTHRITIS PATIENTS TREATED WITH ANTI-TNFALPHA**
ZIRKULIERENDE MIRNAS ALS BIOMARKER DER THERAPIEWIRKSAMKEIT BEI PATIENTEN MIT RHEUMATOIDER ARTHRITIS, DIE MIT ANTI-TNFALPHA BEHANDELT WERDEN
PROCÉDÉ POUR FAIRE CIRCULER DES ARNMI ET TANT QUE BIOMARQUEURS D'EFFICACITÉ D'UNE THÉRAPIE CHEZ LES PATIENTS SOUFFRANT D'ARTHRITE RHUMATOÏDE ET TRAITÉS AVEC ANTI-TNFALPHA

(30) Priority: 22.05.2014 EP 14382178
(43) Date of publication of application: 29.03.2017
(73) Proprietor: Servicio Andaluz de Salud, 41071 Sevilla (ES); Universidad De Córdoba, 14071 Córdoba (ES)
(72) Inventor: LÓPEZ-PEDRERA, Rosario, E-14004 Córdoba (ES); PÉREZ-SÁNCHEZ, Carlos, E-14004 Córdoba (ES); CASTRO VILLEGAS, Carmen, E-14004 Córdoba (ES); LIMÓN-RUIZ, Patricia, E-14004 Córdoba (ES); JIMÉNEZ GÓMEZ, Yolanda, E-14004 Córdoba (ES); COLLANTES ESTÉVEZ, Eduardo, E-14004 Córdoba (ES); BARBARROJA PUERTO, Nuria, E-14071 Córdoba (ES)
(74) Representative: Hoffmann Eitle
(86) International application number: PCT/EP2015/061471
(87) International publication number: WO 2015/177368

(56) References cited:
- WO-A2-2005/118806
- US-A1- 2013 022 985
- FILKOVA M ET AL: "Association of circulating miR-223 and miR-16 with disease activity in patients with early rheumatoid arthritis", ANNALS OF THE RHEUMATIC DISEASES, vol. 73, no. 10, 29 July 2013 (2013-07-29) , pages 1898-1904, XP055212917, ISSN: 0003-4967, DOI: 10.1136/annrheumdis-2012-202815 -& M Filkova ET AL: "Association of circulating miR-223 and miR-16 with disease activity in patients with early rheumatoid arthritis. Supplementary Data.", ANNALS OF THE RHEUMATIC DISEASES, 29 July 2013 (2013-07-29), XP055213072, Retrieved from the Internet: URL:http://ard.bmj.com/content/suppl/2013/ 07/29/annrheumdis-2012-202815.DC1/annrheum dis-2012-202815supp.pdf [retrieved on 2015-09-14]
- PIVARCSI A ET AL: "Changes in the level of serum microRNAs in patients with psoriasis after antitumour necrosis factor-[alpha] therapy", BRITISH JOURNAL OF DERMATOLOGY, vol. 169, no. 3, 30 August 2013 (2013-08-30), pages 563-570, XP055212907, ISSN: 0007-0963, DOI: 10.1111/bjd.12381
- MURATA K ET AL: "Comprehensive microRNA Analysis Identifies miR-24 and miR-125a-5p as Plasma Biomarkers for Rheumatoid Arthritis", PLOS ONE, vol. 8, no. 7, 18 July 2013 (2013-07-18), page e69118, XP055145937, DOI: 10.1371/journal.pone.0069118
- SHIBUYA H ET AL: "Overexpression of microRNA-223 in rheumatoid arthritis synovium controls osteoclast differentiation", MODERN RHEUMATOLOGY, vol. 23, no. 4, 19 August 2012 (2012-08-19), pages 674-685, XP055212929, ISSN: 1439-7595, DOI: 10.3109/s10165-012-0710-1
- CARMEN CASTRO-VILLEGAS ET AL: "Circulating miRNAs as potential biomarkers of therapy effectiveness in rheumatoid arthritis patients treated with anti-TNFalpha", ARTHRITIS RESEARCH AND THERAPY, BIOMED CENTRAL, LONDON, GB, vol. 17, no. 1, 9 March 2015 (2015-03-09), page 49, XP021219893, ISSN: 1478-6354, DOI: 10.1186/S13075-015-0555-Z
- ZHU S ET AL: "The microRNA miR-23b suppresses IL-17-associated autoimmune inflammation by targeting TAB2, TAB3 and IKK-[alpha]", NATURE MEDICINE, vol. 18, no. 7, 3 June 2012 (2012-06-03), pages 1077-1086, XP055084179, ISSN: 1078-8956, DOI: 10.1038/nm.2815
- FULCI V ET AL: "miR-223 is overexpressed in T-lymphocytes of patients affected by rheumatoid arthritis", HUMAN IMMUNOLOGY, NEW YORK, NY, US, vol. 71, no. 2, 1 February 2010 (2010-02-01), pages 206-211, XP026871884, ISSN: 0198-8859 [retrieved on 2009-11-18]
- NAKASA T ET AL: "Expression of microRNA-146 in rheumatoid arthritis synovial tissue", ARTHRITIS & RHEUMATISM, vol. 58, no. 5, 1 May 2008 (2008-05-01), pages 1284-1292, XP055024560, ISSN: 0004-3591, DOI: 10.1002/art.23429
- STANCZYK J ET AL: "Altered expression of microRNA in synovial fibroblasts and synovial tissue in rheumatoid arthritis", ARTHRITIS & RHEUMATISM, WILEY, US, vol. 58, no. 4, 1 April 2008 (2008-04-01), pages 1001-1009, XP002559944, ISSN: 0004-3591, DOI: 10.1002/ART.23386 [retrieved on 2008-03-27]
- LU M-C ET AL: "Increased miR-223 expression in T cells from patients with rheumatoid arthritis leads to decreased insulin-like growth factor-1-mediated interleukin-10 production", CLINICAL & EXPERIMENTAL IMMUNOLOGY, vol. 177, no. 3, 24 September 2014 (2014-09-24), pages 641-651, XP055212901, ISSN: 0009-9104, DOI: 10.1111/cei.12374
- SALEHI E ET AL: "MicroRNAs in rheumatoid arthritis", CLINICAL RHEUMATOLOGY, ACTA MEDICA BELGICA, BRUXELLES, BE, vol. 34, no. 4, 4 March 2015 (2015-03-04), pages 615-628, XP035473180, ISSN: 0770-3198, DOI: 10.1007/S10067-015-2898-X [retrieved on 2015-03-04]
- CHUROV A V ET AL: "MicroRNAs in rheumatoid arthritis: Altered expression and diagnostic potential", AUTOIMMUNITY REVIEWS, vol. 14, no. 11, 8 July 2015 (2015-07-08), pages 1029-1037, XP055212868, ISSN: 1568-9972, DOI: 10.1016/j.autrev.2015.07.005

## Description

The invention relates to the field of medicine, more specifically to the field of medical prognosis, and still more specifically refers to the use of miRNAs for predicting response of a human subject to anti-TNFa therapy, particularly in patients with rheumatoid arthritis.

### Background of the invention

Rheumatoid arthritis (RA) is a systemic, inflammatory, autoimmune disorder of unknown etiology that affects primarily the articular cartilage and bone. Characteristic features of RA pathogenesis are persistent inflammation, synovium hyperplasia and cartilage erosion accompanied by joint swelling and joint destruction (Filková et al., 2012. BioDrugs 1; 26(3), 131-141). Early treatment can prevent severe disability and lead to remarkable patient's benefits, although a lack of therapeutic efficiency in a considerable number of patients remains problematic.

TNFa plays a central role in the pathogenesis of rheumatoid arthritis (RA) and is instrumental in causing joint destruction, the clinical hallmark of the disease. It induces macrophages and other cells to secrete pro-inflammatory cytokines (i.e. IL-1, IL-6 and IL-8), leads to T-cell activation, and induces endothelial cells to express adhesion molecules (Feldmann et al., 1996. Annu. Rev. Immunol. 14, 397-440; Atzeni et al., 2009. Curr. Opin. Investig. Drugs 10(11), 1204-1211). TNFa is involved in the differentiation and maturation of osteoclasts (the main cells involved in arthritic bone destruction), and stimulates fibroblasts, osteoclasts and chondrocytes to release proteinases, which destroy articular cartilage and bone (Feldmann et al., 1996. Annu. Rev. Immunol. 14, 397-440; Choy et al., 2001. N. Engl. J. Med. 22;344(12):907-916; Polachek et al., 2012. Autoimmun. Rev. 12(2):164-8).

The introduction of anti-TNF therapy has significantly improved the outlook for patients suffering from RA. Yet, a substantial proportion of patients fail to respond to these therapies. Treatment response is likely to be multifactorial; however, variation in genes or their expression may identify those most likely to respond (Prajapati et al., 2011. Pharmacogenomics 12(11):1571-1585). By targeted testing of variants within candidate genes, potential predictors of anti-TNF response have been reported (Plenge et al., 2008. Curr. Opin. Rheumatol. 20(2):145-52). However, very few markers have replicated consistently between studies. Other potential serum biomarkers of response have also been explored including cytokines and autoantibodies, with antibodies developing to the anti-TNF drugs themselves being correlated with treatment failure (Choi et al., 2013 Ann. Rheum. Dis. doi: 10.1136/annrheumdis-2013-203923; Krintel et al., 2013 Rheumatology Jul; 52(7):1245-1253; Ortea et al., 2012. J. Proteomics. 21, 77,372-82).

More recently, epigenetic anomalies are emerging as key pathogenic features of RA. The effects of epigenetics in RA range from contributing to complex disease mechanisms to identifying biomarkers for early diagnosis and response to therapy. Key epigenetic areas in RA have been evaluated namely DNA methylation, histone modification, and expression and/or function of microRNAS (Bottini et al., 2013. Curr. Rheumatol. Rep. 15(11), 372). MicroRNAs (miRNAs) are small, non-coding RNAs that, depending upon base pairing to messenger RNA (mRNA) mediate mRNA cleavage, translational repression or mRNA destabilization. miRNAs are involved in crucial cellular processes and their dysregulation has been described in many cell types in different diseases (Filková et al., 2012. BioDrugs 1; 26(3), 131-141). In fact, abnormalities in miRNA expression related to inflammatory cytokines, Th-17 and regulatory T cells as well as B cells have been described in several autoimmune diseases (Ceribelli et al., 2011. Arthritis Res. Ther. 13(4):229). Over the past several years it has become clear that alterations exist in the expression of miRNAs in patients with RA. Increasing number of studies have shown that dysregulation of miRNAs in peripheral blood mononuclear cells (Pauley et al., 2008. Arthritis Res. Ther. 10(4):R101) isolated T lymphocytes (Niimoto et al., 2010. BMC Musculoskelet Disord. 15; 11:209), synovial tissue and synovial fibroblasts -that are considered key effectors cells in joint destruction- (Stanczyk et al., 2008. Arthritis Rheum. 58(4), 1001-1009; Stanczyk et al., 2011. Arthritis Rheum. 63(2), 373-381. Nakamachi *et al.,* 2009. 60(5):1294-304), contributes to inflammation, degradation of extracellular matrix and invasive behaviour of resident cells. Moreover, miRNAs have been demonstrated to be present with altered expression in body fluids such as plasma and synovial fluids in RA patients (Murata et al., 2010. Arthritis Res Ther. 12(3):R86).Thus, synovial fluid and plasma miRNAs have additional potential as diagnostic biomarkers for some autoimmune diseases, as well as a tool for the analysis of their pathogenesis.

Additionally, it has been demonstrated that miRNAs can be aberrantly expressed even in the different stages of RA progression, allowing miRNAs to help understand the pathogenesis of the disease, to act as important biomarkers, and to monitor the disease severity (Ceribelli et al., 2011. Arthritis Res. Ther. 13(4):229). Yet, to date no study has evaluated the changes occurred in the profile of serum miRNAs in RA patients after anti-TNFα therapy. Therefore, to identify possible biomarkers predictive of the therapeutic effect of anti-TNFa drugs in RA, we investigated serum miRNA changes after six months of *in vivo* treatment.

### Brief description of the invention

The present invention concerns the *in vitro* use of SEQ ID NO: 2 (miR-23a) as a biomarker for predicting response of a human subject suffering from rheumatoid arthritis to anti-TNFα therapy, comprising determining the expression level thereof.

The invention also concerns a method of predicting response of a human subject suffering from rheumatoid arthritis to anti-TNFα therapy, comprising using, as an indicator, expression levels of SEQ ID NO: 2 (miR-23a) and obtaining a result of the method by determining in samples obtained from said human subject the changed relative expression between T1 and T2 for SEQ ID NO:2, wherein T1 is understood as prior to subject initiating anti-TNFα therapy and T2 is understood as after the subject has initiated anti-TNFα treatment; and wherein the result is indicative for response if the expression in T2 has increased relative to the expression in T1.

The present invention additionally concerns a method of predicting response of a human subject suffering from rheumatoid arthritis, to anti-TNFα therapy comprising using, as an indicator, expression levels of SEQ ID NO: 2 (miR-23a) in combination with expression levels of SEQ ID NO: 6 (miR-223) and obtaining a result of the method by determining the expression levels at T1 and comparing it with a cutoff value, wherein T1 is understood as prior to the subject initiating anti-TNFα treatment, and wherein the result is indicative for response if the expression of both biomarkers in T1 has increased relative to the cutoff value.

The present invention also concerns a method for allocating a human subject suffering from rheumatoid arthritis in one of two groups, wherein group 1 comprises responders subjects identifiable by the method according to any one of the above methods of predicting response of a human subject suffering from rheumatoid arthritis to anti-TNFα therapy, and wherein group 2 represents the remaining subjects.

The invention moreover concerns a pharmaceutical composition comprising an anti-TNFα agent or TNF inhibitor, for use in a method of treatment of responder patients suffering from rheumatoid arthritis which are identified by the previously recited method for allocating a human subject suffering from rheumatoid arthritis in one of two groups.

Finally, the invention concerns the *in vitro* use of a kit comprising at least one oligonucleotide(s) capable of hybridizing with miRNAs set forth as SEQ ID NOs: 2 under stringent conditions in a method according to any one of the above methods.

What is additionally described in the present disclosure is a method of predicting response of a human subject to anti-TNFa therapy, hereinafter first method of the invention, preferably wherein the subject is suffering from rheumatoid arthritis. The method comprises the detection, in a sample from said subject (preferably in an isolated sample), expression levels of one or more miRNAs. These miRNAs are preferably selected from one or more of the group consisting of the ones set forth by the following: SEQ ID NO: 1 (miR-16); SEQ ID NO: 2 (miR-23a); SEQ ID NO: 3 (miR125b); SEQ ID NO: 4 (miR-126a); SEQ ID NO: 5 (miR-146a) and SEQ ID NO: 6 (miR-223). The inventors have shown that the levels of one or more of these miRNAs can be indicative for non-response, or response of a subject.

The method of determining the result, i.e. the expression level of the miRNA is not particularly limited and may be selected form a gene profiling method, such as a microarray, and/or a method comprising PCR, such as real time PCR; and/or Northern Blot. realReal-time PCR is preferred, and the values with respect to a reference value indicative for non-response can be expressed as -ΔΔCt.

It is possible that at the time point of taking the sample from the human subject, the human subject is (i) treated by anti-TNFa therapy or (ii) is not treated by anti-TNFa therapy, but (ii) is preferred. Thus, the response to anti-TNFa therapy can be predicted. The anti-TNFa therapy the response to which can be predicted preferably comprises administration of infliximab, adalimumab, certolizumab, golimumab, etanercept, or combinations thereof, alone or in combination with a further agent. In particular embodiments, the anti-TNFa therapy may comprise administration of Infliximab, etanercept, or of adalimumab.

In the method of the present disclosure, the expression of the miRNA may be normalized, preferably in relation to the expression of another RNA molecule.

The disclosure also provides a method for allocating a human subject suffering from rheumatoid arthritis in one of two groups, hereinafter second method of the disclosure, wherein group 1 comprises subjects identifiable by the method of the invention; and wherein group 2 represents the remaining subjects. It is possible to provide a customized therapy to an individual, depending on whether the individual is allocated to group 1 or group 2.

Thus, the present disclosure also provides a pharmaceutical composition comprising an anti-TNFa agent or a TNF inhibitor, including or not a further agent, preferably selected from infliximab, adalimumab, certolizumab, golimumab, etanercept, or combinations thereof, for treating a human subject of group 1, wherein in this context group 1 is understood as responding or partially responding patients.

Further, this disclosure provides an antibody for treating a human subject of group 1, wherein in this context group 1 is understood as responding or partially responding patients. The antibody is preferably selected from an anti-TNFα antibody.

The present disclosure also provides a kit comprising at least one oligonucleotide(s) capable of hybridizing with any one or more, preferably two or more, and most preferably all, of the miRNAs set forth as SEQ ID NOs: 1 to 6. It is preferred that said oligonucleotide(s) is capable to do so under stringent conditions. It is also possible that said kit additionally comprises a poly T oligonucleotide (DNA) primer. It is also possible that the oligonucleotide(s) are immobilized in spots on a (preferably solid) surface. In one embodiment thereof, the kit comprises a microarray. The kit may be used and the use is not particularly limited, although use in the method of the disclosure is preferred.

The present disclosure also provides a computer readable storage medium storing a program of instructions executable by a machine to perform the steps of the first and/or the second method of the invention.

The present disclosure also provides a signal transmission arrangement comprising a set of instructions to perform the steps of any of the methods of the disclosure.

### Description of the figures

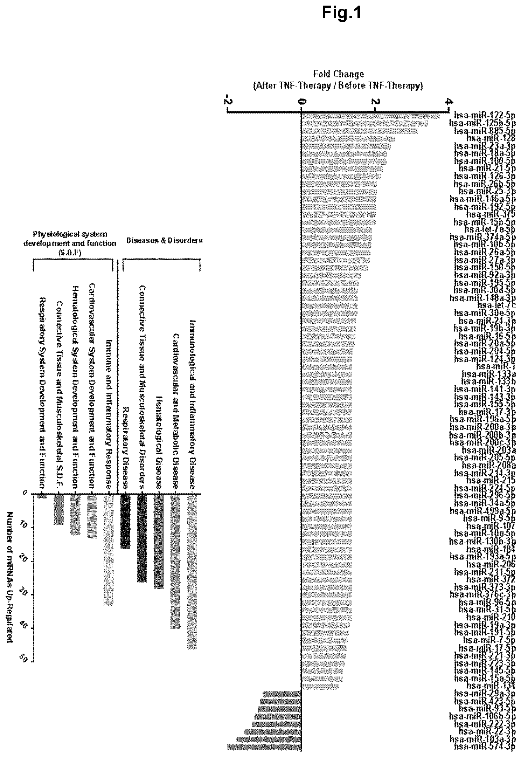

**Figure 1****. Plasma miRNA profiling using miRNA array.** A) To identify the changes occurred in the expression levels of miRNAs in serum from patients treated with anti-TNFa drugs, Human Serum & Plasma miRNA PCR Array (Qiagen) was performed. A pool with 2 µl from RNA purified of 10 patients before treatment, and other pool with 2 µl from RNA purified of the same 10 patients after treatment was performed. The expression levels of miRNAs were normalized to the mean of spiked- in miRNA Cel-miR-39 and was calculated using the 2-ΔΔCt method. B) Differentially expressed genes were classified and used for computational analysis to identify potential functional pathways and networks using the Ingenuity Pathways Analysis Knowledge Base (Ingenuity Systems). The functional analysis identified those biologic functions that were most significant for the data set.

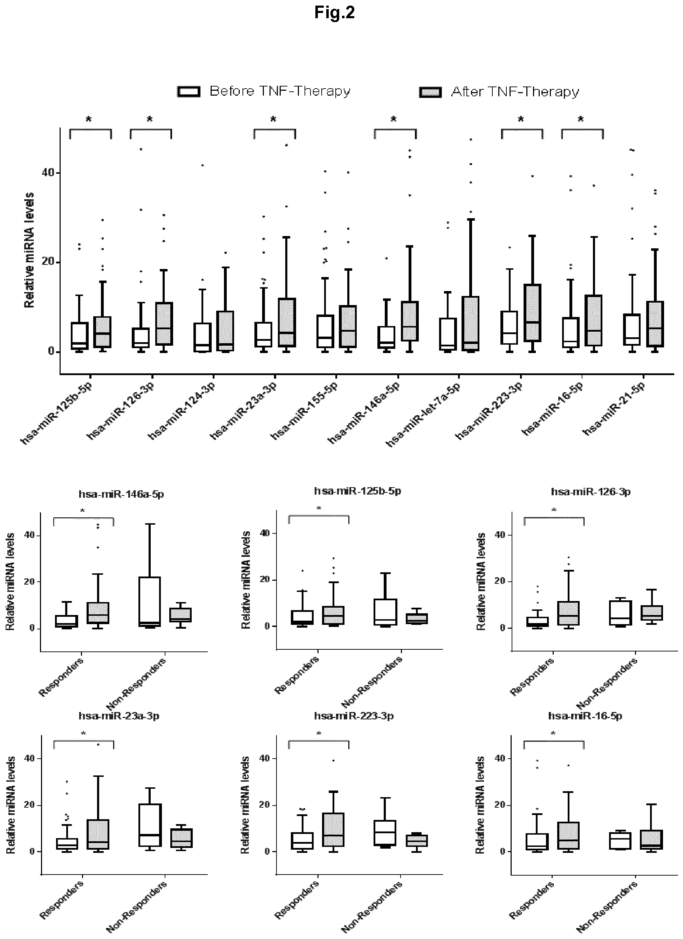

**Figure 2****. Relative miRNA levels at starting (T1) and after six month of anti-TNFα therapy (T2) in the validation cohort (n=85).** To validate the PCR Array data, ten microRNAs differentially expressed were selected (hsa-miR-125b, hsa-miR-23a-3p, hsa-miR-21-5p, hsa-miR-126-3p, hsa-miR-146a-5p, hsa-let-7a-5p, hsa-miR-16-5p, hsa-miR-124a-3p, hsa-miR-155-5p, and has-miR-223). A) Relative expression levels of each miRNA are shown. Boxes indicate the interval between the 25^{th} and 75^{th} percentiles and horizontal bars inside boxes indicate median. Whiskers indicate the interval of data within 1.5xinterquartile ranges (IQR). Closed circles indicate data points outside 1.5xIQR. *P<0.05. B) Comparison of relative change of miRNA levels between Responders and Non-Responders groups for the six miRNAs found significantly altered after therapy in the validation cohort.*P<0.05.

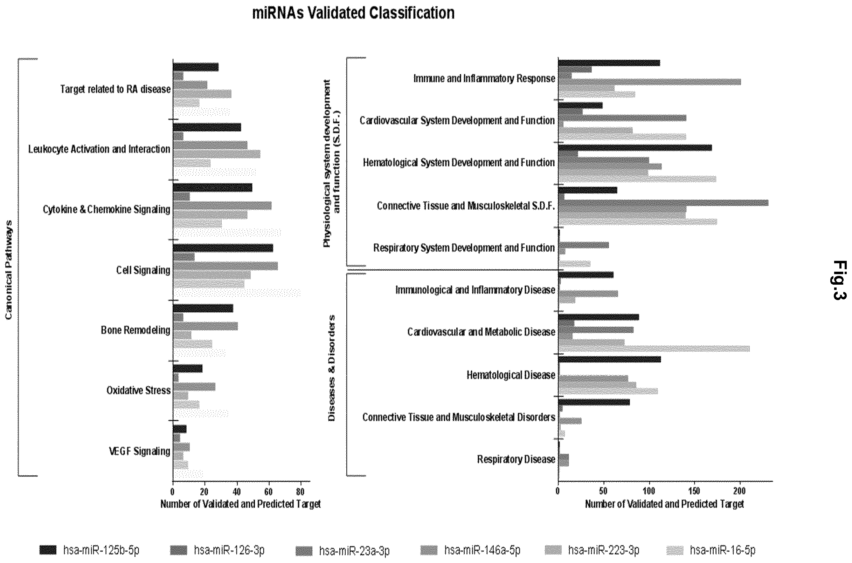

**Figure 3****. Biological categories of validated miRNA and predicted functions.** By using the IPA analysis, gene targets predictions for the six validated miRNAs were performed. Ingenuity Pathways uncovered the main enriched biological pathways (A), as well as the main biofunctions and disorders on which that miRNAs are involved (B).

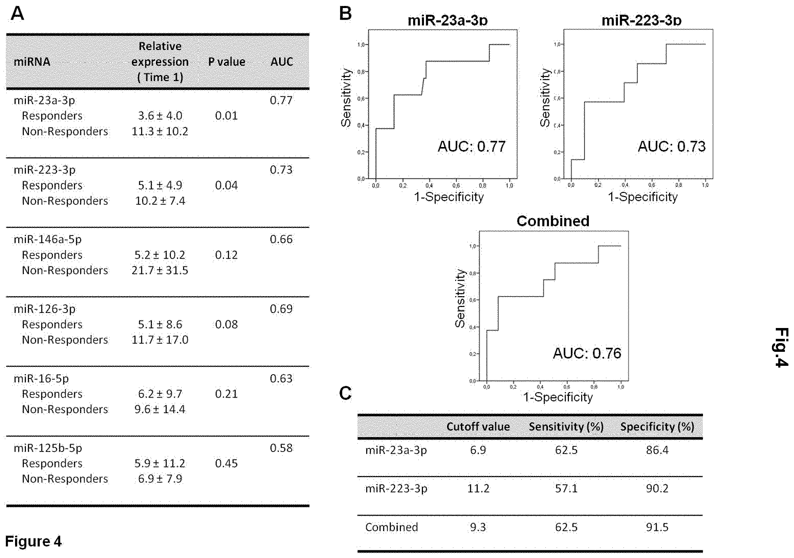

**Figure 4****. Evaluation of candidate miRNAs as predictors of response to therapy.** A. Relative expression levels of the six miRNAs validated in plasma of RA patients (n=95) before starting the anti-TNF therapy (T1). Data are shown as mean ± standard deviation. Area under curve (AUC) was calculated after plotting receiver operating characteristic (ROC) curve. B. ROC curve analyses of miR-23a-3p (left panel) and miR-223-3p (right panel), which showed the highest values for AUC; below is shown the combined panel for the two miRNAs performed as described in material and methods. C. Sensitivity and specificity of each miRNA test. Cutoff value with higher specificity was selected.

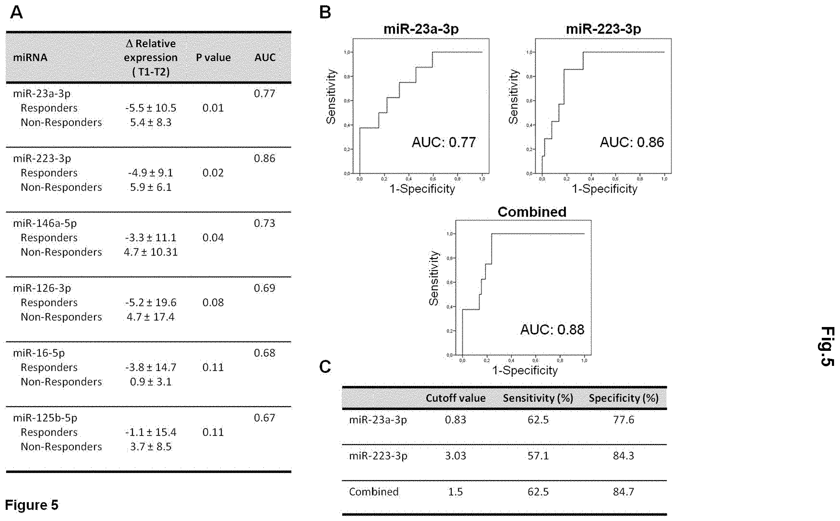

**Figure 5****. Evaluation of candidate miRNAs as potential biomarkers of response to anti-TNF therapy.** A. Changes in relative expression levels of the six miRNAs validated in plasma of RA patients (n=95) before and after anti-TNF therapy (T1-T2). Data are shown as mean ± standard deviation. Area under curve (AUC) was calculated after plotting receiver operating characteristic (ROC) curve. B. ROC curve analyses of miR-23a-3p (left panel) and miR-223-3p (right panel), which showed the highest values for AUC; below is shown the combined panel for the two miRNAs performed as described in material and methods. C. Sensitivity and specificity of each miRNA test. Cutoff value with higher specificity was selected.

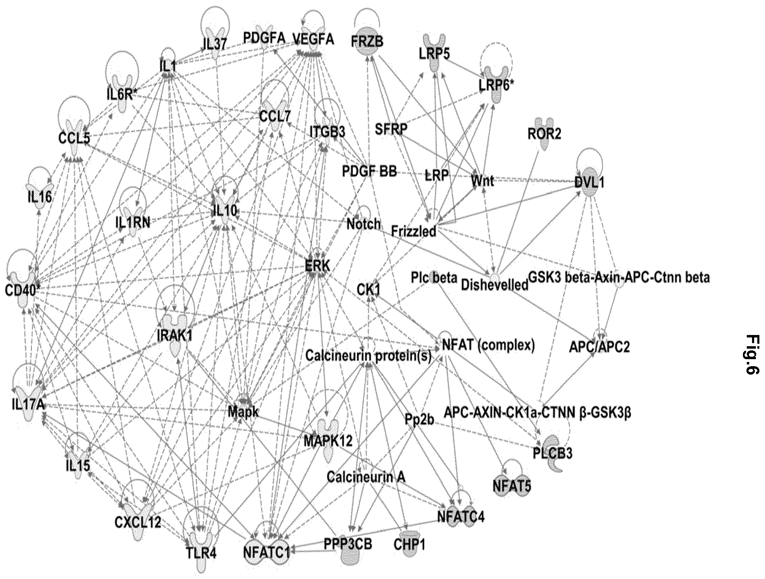

**Figure 6****. Pathway analysis and networking of miRNA target genes.** Gene networks showing inter-relationship between targets of the differentially expressed miRNAs using IPA analysis software. The genes indicated in green are related to inflammatory pathways and the genes indicated in orange are mainly associated to bone remodelling pathways. Direct interactions appear in the network diagram as a solid line, whereas indirect interactions as a dashed line.

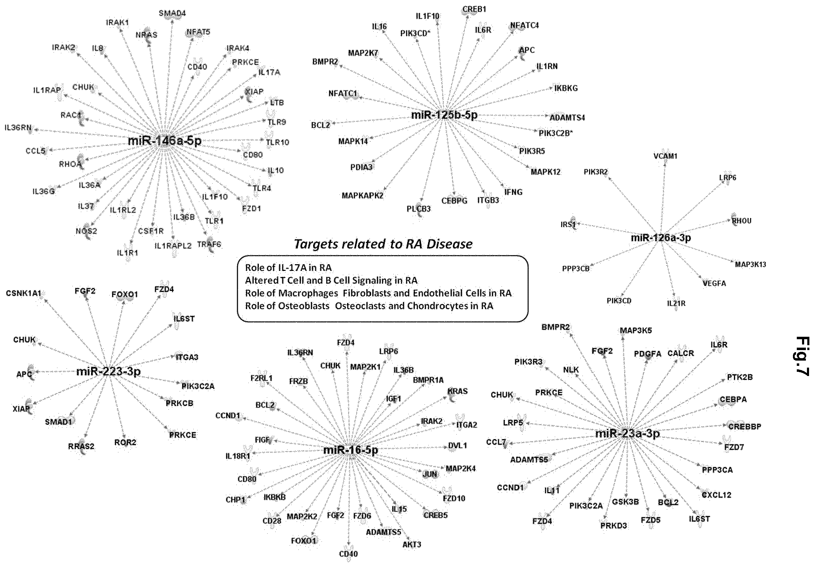

**Figure 7****. Network of validated microRNAs and their target genes associated to Rheumatoid Arthritis disease.** Interestingly, a number of both miRNA and mRNA targets uncovered in the network, were found complementary altered after anti-TNF treatment in our patient's cohort.

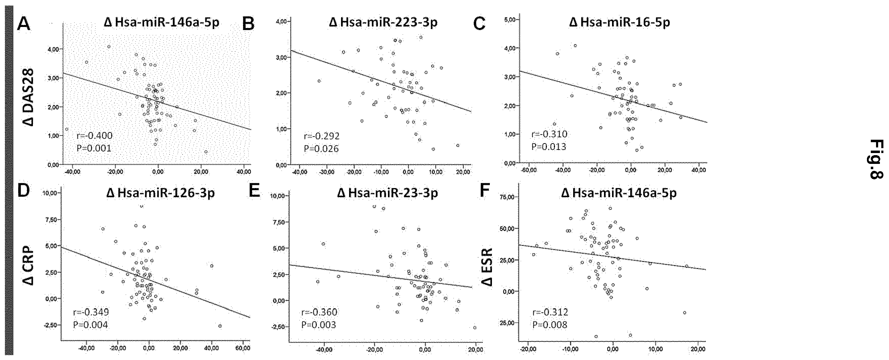

**Figure 8****. Changes in serum miRNAs correlate with changes in clinical variables in RA patients**
   Changes after anti-TNF therapy (T1-T2) of various miRNAs significantly correlated with the changes in DAS28 (A-C), in CRP (D-E) and in ESR (F). r values of Spearman's rank correlation and P-values of their null hypothesis are shown.

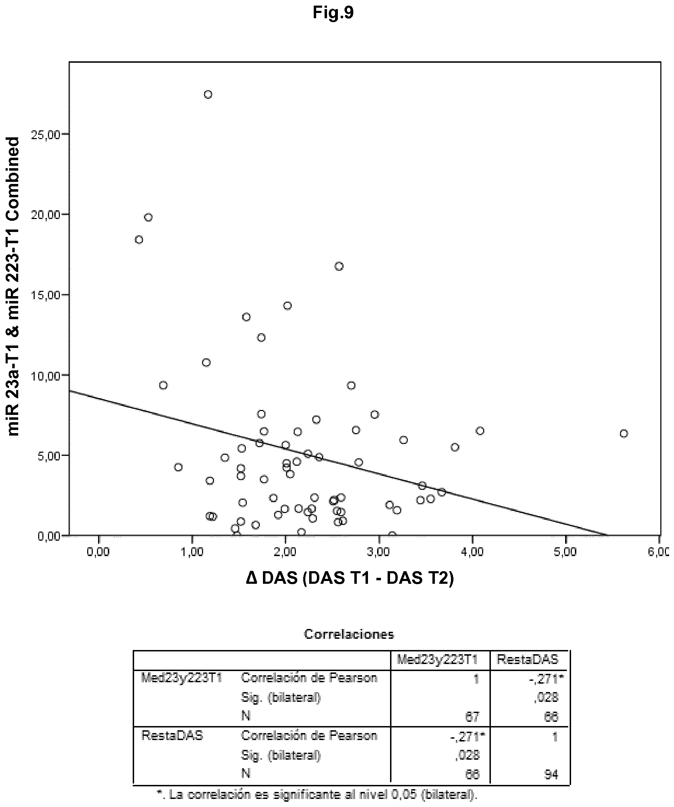

**Figure 9****. Combined expression levels of serum miRNAs 23a and 223 at T1 correlated with the changes occurred in DAS after anti-TNFa treatment. R** values of Pearson's rank correlation and P-values are shown in the table.

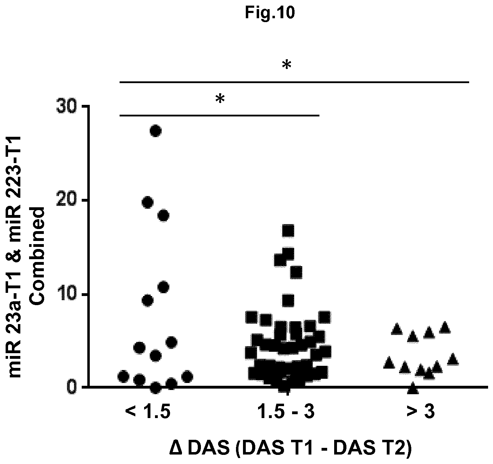

**Figure 10****. Combined expression levels of serum miRNAs 23a and 223 at T1 can predict the degree of clinical response.** Stratification of patients in three groups depending on the degree of DAS changes (change units less than 1,5, between 1.5 and 3 and more than 3) showed that there is an specific range of combined expression of the miRNas 23a and 223 at T1, which can predict each range of response.

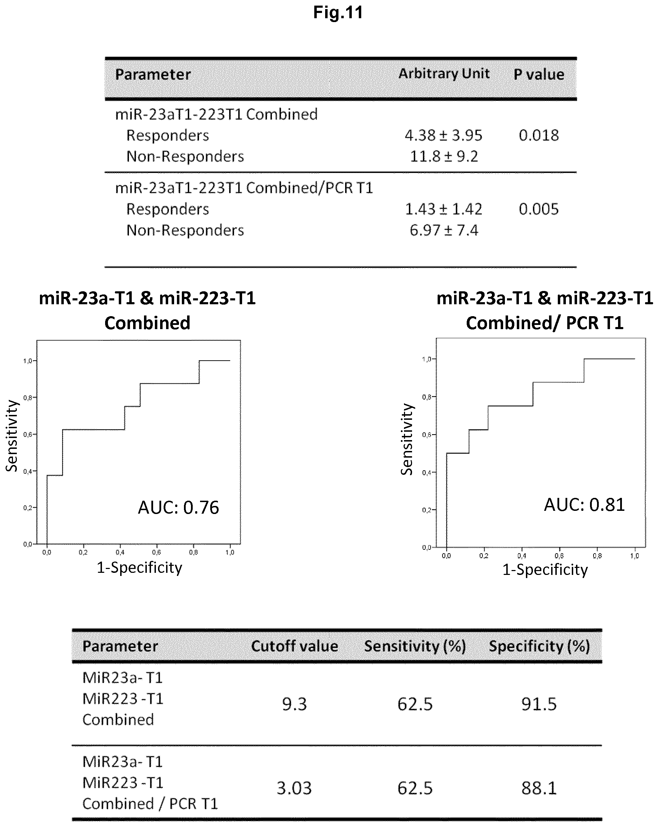

**Figure 11****. Combined microRNA 23a and 223 levels and an additional clinical parameter such as CRP at T1 migth also help to discriminate responders from non-responders patients.** Relative expression levels of miRNAs 23a and 223 plus CRP levels in plasma of RA patients (n=95) before starting the anti-TNFa/DMARDs combination therapy (T1). Data are shown as mean ± standard deviation. Area under curve (AUC) was calculated after plotting receiver operating characteristic (ROC) curve. ROC curve analyses of combined miR-23a and miR223 (left panel) and combined miR-23 and miR 223/CRP ratio (right panel), which showed the highest values for AUC; Sensitivity and specificity of each parameter are shown in the table. Cutoff value with higher specificity was selected.

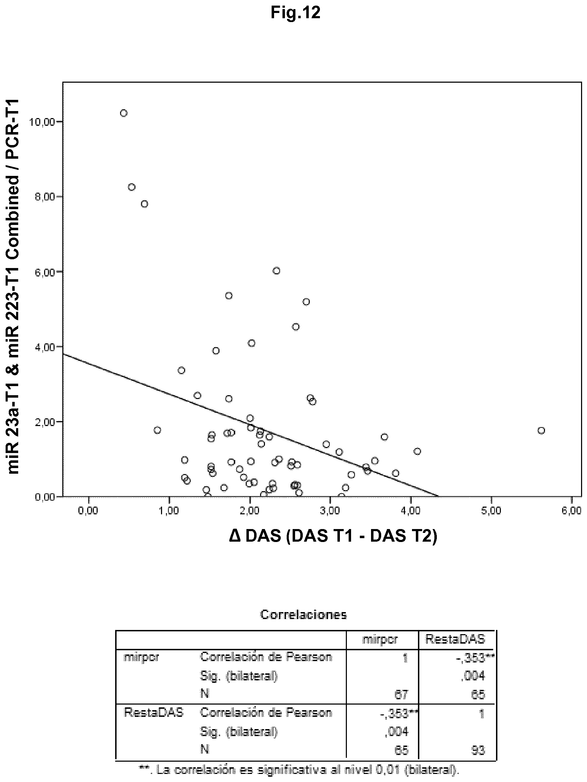

**Figure 12****.** Combined expression levels of serum miRNAs 23a and 223/CRP ratio at T1 correlated with the changes occurred in DAS after anti-TNFa treatment. R values of Pearson's rank correlation and P-values are shown in the table.

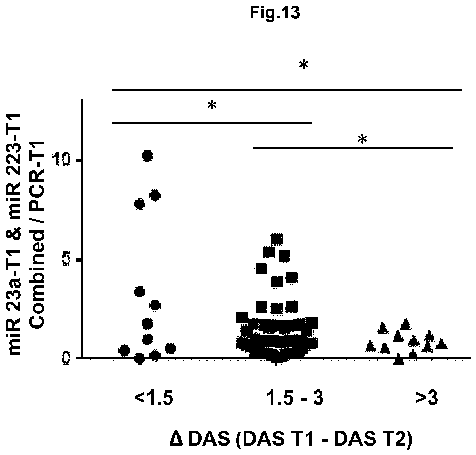

**Figure 13****. Combined expression levels of serum miRNAs 23a and 223/CRP ratio at T1 can predict the degree of clinical response.** Stratification of patients in three groups depending on the degree of DAS changes (change units less than 1,5, between 1.5 and 3 and more than 3) showed that there is an specific range of combined expression of the miRNas 23a and 223/CRP ratio at T1, which can predict each range of response.

### Detailed description of the invention

The detailed description discloses specific and/or preferred variants of the individual features of the invention. The present invention is defined in the appended claims.

Provided in the present disclosure is a method of predicting response of a human subject to anti-TNFα therapy, hereinafter first method of the invention, preferably wherein the subject is suffering from an autoimmune disease, and more preferably suffering from rheumatoid arthritis. The method comprises the detection, in a sample from said subject (preferably an isolated sample), expression levels of one or more miRNAs. These miRNAs are one or more selected from the group consisting of the ones set forth by the following: SEQ ID NO: 1 (miR-16); SEQ ID NO: 2 (miR-23a); SEQ ID NO: 3 (miR125b); SEQ ID NO: 4 (miR-126a); SEQ ID NO: 5 (miR-146a) and SEQ ID NO: 6 (miR-223). The inventors have shown that the levels of one or more of these miRNAs can be indicative for non-response, or response of a subject.

The terms "human subject" "subject" and "patient" are therefore used interchangeably in this specification.

One or more" also as used herein includes one and the individualized specification of any number which is more than one, such as two, three, four, five, six etc. "More than one" or "several" as used herein includes the individualized specification of any number which is more than one, such as two, three, four, five, six etc.

The method of the disclosure comprises the detection, in a sample from a human subject, of the expression levels of one or more particular miRNAs. Illustrative nonlimiting examples of said sample include different types of samples from tissues, as well as from biological fluids, such as blood, serum, plasma, cerebrospinal fluid, peritoneal fluid, faeces. Preferably, said samples are whole blood samples, and more preferably said samples are serum and/or plasma.

MicroRNAs are ∼22-nucleotide (approx. 18-25 nucleotides) single-stranded RNAs and negatively regulate (inhibit) gene expression through translational inhibition or mRNA cleavage. Thus, miRNAs are post-transcriptional regulators that bind to complementary sequences on target messenger RNA transcripts (mRNAs), usually resulting in translational repression or target degradation and gene silencing. In a particular aspect of the invention, understanding the molecular details of action of the miRNAs of the invention is not critical, since the detected levels of the indicative miRNAs alone allow for performing the methods of the invention.

In particular, the present disclosure relates to a method involving a set consisting of miRNAs as follows: SEQ ID NO: 1 (miR-16); SEQ ID NO: 2 (miR-23a); SEQ ID NO: 3 (miR125b); SEQ ID NO: 4 (miR-126a); SEQ ID NO: 5 (miR-146a); SEQ ID NO: 6 (miR-223).

**Table III: Sequences of miRNAs**

| **MicroRNA** | **SEQ ID NO** | **Sanger MatureID** | **Mature Sequence** |
|---|---|---|---|
| hsa-miR-16-5p | 1 | MIMAT0000069 | uagcagcacguaaauauu ggcg |
| hsa-miR-23a-3p | 2 | MIMAT0000078 | aucacauugccagggauu ucc |
| hsa-miR-125b-5p | 3 | MIMAT0000423 | ucccugagacccuaacuug uga |
| hsa-miR-126-3p | 4 | MIMAT0000445 | ucguaccgugaguaauaa ugcg |
| hsa-miR-146a-5p | 5 | MIMAT0000449 | ugagaacugaauuccaug gguu |
| hsa-miR-223-3p | 6 | MIMAT0000280 | ugucaguuugucaaauac ccca |

Under a standard nomenclature system, names are assigned to experimentally confirmed miRNAs as follows: the prefix "mir" is followed by a dash and a number, whereby the latter may indicate the order of naming. The un-capitalized "mir-" refers to the pre-miRNA, while a capitalized "miR-" refers to the mature form. miRNAs with nearly identical sequences, except one or two nucleotides, are annotated with an additional lower case letter, for example miR99a*. Pre-miRNAs that lead to 100% identical mature miRNAs but that are located at different places in the genome are indicated with an additional dash-number suffix. Species of origin may be designated with a three-letter prefix, e.g., hsa-miR-223 is a human (Homo sapiens) miRNA. Since in the context of this document, all individualised miRNAs are human miRNAs, the prefix "hsa-" is sometimes omitted. When two mature microRNAs originate from opposite arms of the same pre-miRNA, they are denoted with a -3p or -5p suffix, such as for example miR-126-3p. When relative expression levels are known, an asterisk following the name indicates an miRNA expressed at low levels relative to the miRNA in the opposite arm of a hairpin. The majority of known miRNA genes is found in intergenic regions or oriented antisense to neighboring genes and is therefore thought to be transcribed as independent units. Their genes are usually transcribed by RNA polymerase II, and the transcripts processed, exported from the nucleus and further processed by specific machineries, as is well known in the art (see e.g. He et al., Nat. Rev. Genet. 2004 Jul;5(7):522-31). miRNA sequences can be accessed at http://www.mirbase.org.

The fact that the present invention uses miRNAs (i.e. very short and well-defined RNAs) as indicative factors comes with the advantage of easy and reliable detection in contrary to some known methods, such as the use of messenger RNAs (mRNAs). mRNA molecules are typically several hundred or even several thousand bases in size; such large and at the same time delicate molecules are generally prone to degradation, and their detection depends on the (oftentimes trial-and error) choice of the appropriate probe, both of which renders such tests prima facie less reliable than the methods of the present invention.

The miRNAs indicative in the method of the present disclosure are one or more selected from the group consisting of the ones set forth by the following SEQ ID NOs: SEQ ID NO: 1 (miR-16); SEQ ID NO: 2 (miR-23a); SEQ ID NO: 3 (miR125b); SEQ ID NO: 4 (miR-126a); SEQ ID NO: 5 (miR-146a) and SEQ ID NO: 6 (miR-223). The inventors have provided evidence that, these miRNAs are indicative of a response of the subject (see Examples).

These six miRNAs for validation were found significantly up-regulated by anti-TNFa treatment (miR-16, miR-23a, miR125b, miR-126a, miRN-146a, miR-223). Responder's patients showed a stronger increase in those miRNAs than no responders, in parallel with the reduction of TNFa, IL6, IL17, RF and CRP. Correlation studies demonstrated multiple associations between validated miRNAs and clinical and inflammatory parameters.

The method involves comparison of said miRNA levels with levels of said miRNAs from a reference sample or with median value. In the context of the present invention, "reference sample" is understood as the reference sample which is used to determine the variation of the expression levels of the miRNA genes of the present invention. In an embodiment, the reference value is obtained from the provided signal using a sample obtained from an individual who is a non-responder.

Preferably, (reference) samples are taken from several non-responder individuals and combined, such that the reference value of reflects the mean value of said molecules in the population of non-responders. "Reference value" is the expression level of a miRNA of the invention in reference sample

Among those miRNAS, we developed a model that identified a specific plasma signature (miR-23 and miR-223) that may serve both as predictors of therapy response and as biomarkers of response to anti-TNF treatment.

Then, in a preferred embodiment, the present invention relates to a method involving a set consisting of miRNAs as follows: SEQ ID NO: 2 (miR-23a) and SEQ ID NO: 6 (miR-223).

Thus, by the method of the invention, an individual patient can be predicted to show either (i) response (R) to anti-TNF treatment or (ii) non-response (NR) to anti-TNF treatment.

The inventors have shown that expression levels of one or more miRNAs as set forth by SEQ ID NOs: 1, 2, 3, 4, 5, 6 are indicative, and in particular, that the result of the method is indicative of a response or a non-response if either one or more of the following are observed: (i) the levels of the miRNA as set forth by SEQ ID NO: 1, SEQ ID NO: 2, SEQ ID NO: 3, SEQ ID NO: 4, SEQ ID NO: 5 and/ or SEQ ID NO: 6 are increased or decreased as detailed in the examples and figures.

In the invention, the method of determining the result, i.e. the expression level of the miRNA need not be particularly limited, and may be selected form a gene profiling method, such as a microarray, and/or a method comprising PCR, such as real time PCR; and/or Northern Blot. Real-time quantitative PCR (RQ-PCR) is a sensitive and reproducible gene expression quantification technique which can particularly be used to profile miRNA expression in cells and tissues. Any method for evaluation of RT-PCR results may be used, and the ΔΔCt-method may be preferred. The ΔΔCt-method is described in detail by Livak et al. (Methods 2001, 25:402-408). (Ct = Cycle threshold values). When putting the present invention into practice, the ΔΔCt-method as described by Livak et al. (Methods 2001, 25:402-408) shall preferably be used. The ΔΔCt-method will involve a 'control sample' and a 'subject sample'. The 'subject sample' is a sample from the subject to be analyzed. For each sample, a target gene (here: miRNA of interest) and an endogenous control gene (as described below) are included for PCR amplification from (typically serially diluted) aliquots. Typically several replicates are used for each diluted concentration to derive amplification efficiency. PCR amplification efficiency can be defined as percentage amplification (from 0 to 1). During the qPCR reaction, a software typically measures for each sample the cycle number at which the fluorescence (indicator of PCR amplification) crosses an arbitrary line, the threshold. This crossing point is the Ct value.

A microarray is an array on a solid substrate (usually a glass slide or silicon thin-film cell) that assays large amounts of biological material, in the present case large amount of different miRNAs or, preferably, their reverse DNA transcripts, which are detectable via specific probes immobilized on the solid substrate.

A Northern Blot involves the use of electrophoresis to separate RNA samples by size and subsequent detection with a hybridization probe complementary to (part of) the target sequence of the RNA of interest.

It is possible that at the time point of taking the sample from the human subject, the human subject is (i) treated by anti-TNFa therapy or (ii) is not treated by anti-TNFa therapy, but (ii) is preferred. Thus, the response to anti-TNFa therapy can be predicted. The anti-TNFa therapy the response to which can be predicted preferably comprises administration of infliximab, adalimumab, certolizumab, golimumab, etanercept, or combinations thereof, alone or in combination with a further agent. In particular embodiments, the anti-TNFa therapy may comprise administration of Infliximab, etanercept, or of adalimumab.

In the context of this specification, the term "treatment" or "treating" means the administration of an agent to prevent, relieve or eliminate one or more symptoms associated with the autoimmune disease, and more preferably with the rheumatoid arthritis. "Treatment" also includes preventing, relieving or eliminating the physiological sequelae of the disease. In the context of this invention, the term "relieve" is understood to mean any improvement of the situation of the treated patient - both subjectively (feelings of or about the patient) and objectively (measured parameters).

Anti-TNFa therapy or anti-TNFa treatment refers to the use of drugs or substances that suppresses response to tumor necrosis factor (TNF), which is part of the inflammatory response. TNF is involved in clinical problems associated with autoimmune disorders such as rheumatoid arthritis, ankylosing spondylitis, Crohn's disease, psoriasis, hidradenitis suppurativa and refractory asthma, so a TNF inhibitor may be used in their treatment. This inhibition can be achieved with a monoclonal antibody such as infliximab (Remicade), adalimumab (Humira), certolizumab pegol (Cimzia), and golimumab (Simponi), or with a circulating receptor fusion protein such as etanercept (Enbrel). While most clinically useful TNF inhibitors are monoclonal antibodies, some are simple molecules such as xanthine derivatives (e.g. pentoxifylline) and Bupropion. Bupropion is the active ingredient in the smoking cessation aid Zyban and the antidepressant Wellbutrin. Several 5-HT2A agonist hallucinogens including (R)-DOI, TCB-2, LSD and LA-SS-Az have also been found to act as potent inhibitors of TNF, with DOI being the most active, showing TNF inhibition in the picomolar range, an order of magnitude more potent than its action as a hallucinogen.

The method of the present disclosure may be applied with samples from individuals of either sex, i.e. men or women, and at any age.

In the method of the present disclosure, the expression of the miRNA may be normalized, preferably in relation to the expression of another RNA molecule. There are well-known normalization methods in the state of the art.

In another aspect, the disclosure also provides a method for allocating a human subject suffering from an autoimmune disease, and preferably suffering from rheumatoid arthritis, in one of two groups, hereinafter second method of the invention, wherein group 1 comprises subjects identifiable by the method of the disclosure as respondents as described in detailed in the figures and examples; and wherein group 2 represents the remaining subjects. It is possible to provide a customized therapy to an individual, depending on whether the individual is allocated to group 1 or group 2. Group 1 comprises subjects identifiable by the method of the invention as: responsive, partially responsive, completely responsive; and wherein group 2 represents the remaining subjects. It is possible to provide a customized therapy to an individual, depending on whether the individual is allocated to group 1 or group 2.

The present inventors have thus identified a novel subgroup of patients that will profit from anti-TNF therapy. Thus, the present disclosure also provides a pharmaceutical composition comprising an anti-TNFa agent, including or not a further agent, preferably selected from infliximab, adalimumab, certolizumab, golimumab, etanercept, or combinations thereof, for treating a human subject of group 1. Given the positive prediction, the disclosure thus provides a method for selecting patients (group 1) which can particularly profit from administration of such a therapy.

Further, the present disclosure provides an antibody for treating a human subject of group 1. The antibody is preferably selected from an anti-TNFα antibody.

The present disclosure also discloses a kit comprising at least one oligonucleotide(s) capable of hybridizing with any one or more, preferably two or more, and most preferably all, of the miRNAs set forth as SEQ ID NOs: 1 to 6. It is preferred that said oligonucleotide(s) is capable to do so under stringent conditions. In a preferred aspect one or more of said one or more (preferably DNA) oligonucleotide(s) are further defined by the following TAqMan® sequences:
- TaqMan® Assays 4427975; 000391
- TaqMan® Assays 4427975; 000399
- TaqMan® Assays 4427975; 000449
- TaqMan® Assays 4427975; 002228
- TaqMan® Assays 4427975; 000468
- TaqMan® Assays 4427975; 002295
respectively.

Stringency is a term used in hybridization experiments. Stringency reflects the degree of complementarity between the oligonucleotide and the nucleic acid (which is in this case the miRNA to be detected); the higher the stringency, the higher percent homology between the probe and filter bound nucleic acid. It is well known to the skilled person that the temperature and salt concentrations have a direct effect upon the results that are obtained. It is recognized that the hybridization results are related to the number of degrees below the Tm (melting temperature) of DNA at which the experiment is performed. Often, stringent conditions are defined as a wash with 0.1X SSC (saline-sodium citrate (SSC) buffer at 65°C. (SSC is typically provided as 20X stock solution, which consists of 3 M sodium chloride and 300 mM trisodium citrate (adjusted to pH 7.0 with HCl)).

In particular aspects, the kit is selected from (a) a kit suitable for PCR, (b) a kit suitable for Northern Blot and (c) a kit suitable for microarray analysis. Any two or more of these embodiments may also be combined, so that the kit may comprise, for example both (a) and (c).

In the case of (a) a kit suitable for PCR, this PCR is typically real-time quantitative PCR (RQ-PCR), a sensitive and reproducible gene expression quantification technique. In this case it is desired that the kit additionally comprises a polyT oligonucleotide primer in addition to the oligonucleotide(s) of the kit. The polyT oligonucleotide primer may be used together with the oligonucleotide(s) of the disclosure for priming PCR, following polyadenylation of the isolated miRNAs by methods known to the skilled person, such as using poly(A) polymerase and ATP. These reagents may optionally be comprised within the kit.

A Northern Blot involves the use of electrophoresis to separate RNA samples by size and subsequent detection with an oligonucleotide(s) (hybridization probe) complementary to (part of) the target sequence of the RNA of interest.

It is also possible that the oligonucleotide(s) are immobilized in spots on a (preferably solid) surface. In one embodiment thereof, the kit comprises a microarray. An RNA microarray is an array on a solid substrate (usually a glass slide or silicon thin-film cell) that assays large amounts of different RNAs (here miRNAs), which are detectable via specific probes immobilized on spots on the solid substrate. Each spot contains a specific nucleic acid sequence, typically a DNA sequence, known as probes (or reporters). While the number of spots is not as such limited, there is a preferred embodiment in which the microarray is customized to the methods of the invention. In one embodiment, such a customized microarray comprises fifty spots or less, such as thirty spots or less, including twenty spots or less.

The kit may be used and the use is not particularly limited, although use in the method of the invention in any of its embodiments is preferred.

The present disclosure also provides a computer readable storage medium storing a program of instructions executable by a machine to perform the steps of the first and/or the second method of the invention.

Lastly, the present disclosure relates to the, *in vitro,* use of the expression levels of SEQ ID NO: 2 (miR-23a) and/or SEQ ID NO: 6 (miR-223) as biomarkers for predicting response of a human subject suffering from rheumatoid arthritis to anti-TNFα therapy, comprising determining the expression level thereof.

Preferably said use further comprises using, as biomarkers, the expression levels of one or more miRNAs as set forth by the following SEQ ID NOs:
(i) SEQ ID NO: 1 (miR-16);
(ii) SEQ ID NO: 3 (miR125b);
(iii) SEQ ID NO: 4 (miR-126a);
(iv) SEQ ID NO: 5 (miRN-146a).

An additional aspect of this disclosure refers to a bio-signature suitable for predicting response of a human subject suffering from rheumatoid arthritis to anti-TNFa therapy comprising the expression levels of SEQ ID NO: 2 (miR-23a) and/or SEQ ID NO: 6 (miR-223). Preferably further comprising the expression levels of one or more of the miRNAs set forth in the precedent paragraph.

The present disclosure also provides a signal transmission arrangement comprising a set of instructions to perform the steps of any of the methods of the invention.

### Examples of the invention

### Patients and Methods

### Patients

Ninety five RA patients were included in the study (during a period of 24 months) after ethics committee approval was obtained. All the RA patients fulfilled the American College of Rheumatology criteria for the classification of RA (Aletaha et al., 2010. Arthritis Rheum. 62(9), 2569-2581). Patients provided written informed consent.

All patients had inadequate response to at least two disease modifying anti-rheumatic drugs (DMARDs), one of which was metotrexate. Patients received DMARDs in monotherapy or in combination therapy. Only patients who were naïve to anti-TNFa agents were included in the study. Therapy with anti-TNFa agents was stable during the study and was associated to DMARDs. Within the cohort, 55 patients were given Infliximab (IFX; 3mg/kg/day intravenous infusion at times 0, 2 and 6 weeks, and every 8 weeks thereafter); 25 received etanercept (ETA, 25 mg subcutaneously twice weekly), and 15 adalimumab (ADA; 40mg subcutaneously every week) for six months. Blood samples were obtained before the starting and at the end of the treatment. Clinical and laboratory parameters of the RA patients included in that treatment protocols are displayed in Table 1.

Patients were evaluated clinically and analytically at baseline (T1) and 6 months of treatment (T2). Clinical assessment included swollen joint count (SJC), tender joint count (TJC), visual analogue scale of pain (VAS; range 1-100 mm) of patient and clinician, SDAI (simple disease activity index), HAQ (health assessment questionnaire) and number of DMARDs associated with anti-TNF treatment. Serological evaluation included analysis of rheumatoid factor (RF), antibodies anti-cyclic citrullinated peptides (anti-CCPs), C-reactive protein (CRP, mg/L) and erythrocyte sedimentation rate (ESR, mm/h).

Response to anti-TNFa treatment was assessed by the 28-joint disease activity score (DAS28). The patients were categorized into responders and non-responders based on the change in the DAS28 score. An improvement in DAS28 over > 1.2 was considered a good response; a reduction in DAS28 of < 1.2 was considered a non-response.

### Blood sample collection and assessment of biological parameters

Whole blood from subjects was collected by direct venous puncture into tubes with Ethylenediaminetetra-acetic acid (EDTA) as an anticoagulant. All the blood was processed for the isolation of plasma within 4 h of collection. The blood was processed by spinning at 2000 X g for 10 min at room temperature. Then, plasma was transferred to a fresh RNase free tube and stored at -80°C. RF was measured by immunoturbidimetric assay (Qantia RF kit, ABBOT Laboratories, Illinois, USA) and concentrations >30 IU/ml were considered positive. Determination of anti-cyclin citrulinated peptide (anti-CCP) antibody was tested with the EDIA™ anti-CCP kit (Euro Diagnostica, Malmö SWEDEN). Positive anti-CCP titers were considered at a concentration of >5 U/mL.

**Table 1. Clinical characteristics of Rheumatoid Arthritis patients recruited to the study**

| | **Exploratory Cohort (n=10)** | **Validation Cohort (n=85)** |
|---|---|---|
| **Sex (Male/Female)** | 1/9 | 11/74 |
| **Age, mean (range)** | 54.6 (38-74) | 53.6 (24-72) |
| **Disease duration (y), mean (range)** | 10.1 (2-23) | 10.4 (1-36) |
| **Smoking, number (%)** | 4(40%) | 23 (27.1%) |
| **TJC, mean** | 13.7±5.8 | 15.7±4.6 |
| **SJC, mean** | 16.9±6.5 | 11.5±3.7 |
| **DAS28, mean** | 5.9±0.7 | 5.7±0.6 |
| **SDAI** | | |
| **HAQ** | 2.14±0.5 | 2.1±0.3 |
| **ESR (mm), mean** | 55±18.62 | 55.9±16.6 |
| **CRP (mg/dL), mean** | 3.6±1.12 | 3.8±2.1 |
| **Positive Rheumatoid Factor, n(%)** | 7(70%) | 60(70.6%) |
| **Positive anti-CCP antibody, n(%)** | 4(40%) | 59(69.4%) |
| **Medication, n(%)** | | |
| **Infliximab** | 9(90%) | 46(54.1%) |
| **Etanercept** | 1(10%) | 24(28.2%) |
| **Adalimumab** | 0 | 15(17.6%) |
| **Corticoids, n(%)** | 4(40%) | 57 (67.1%) |
| **Hidroxichloroquine, n(%)** | 5(50%) | 41 (48.2%) |
| **Azathioprine, n(%)** | 1 (90%) | 9 (10.6%) |
| **Metotrexate, n(%)** | 10 (100%) | 85 (100%) |
| **Sulfasalazine, n(%)** | 6 (60%) | 54 (63.5%) |
| **Cyclosporine, n(%)** | 1 (10%) | 4 (4.7%) |
| **Leflunomide, n(%)** | 6 (60%) | 57 (67.1%) |

| | | |
|---|---|---|
| *TJC*=Tender Joint Count, *SJ*= Swolen Joint Count, DAS28=*Disease Activity Score, SDAI*= *Simplified Disease Activity Index*, *HAQ*= *Health Assessment Questionnaire,* ESR= Erythrocyte sedimentation rate, CRP= C-reactive protein. RF=Reumatoid Factor, Anti-CCP=Anti-cyclic Citrullinated Peptide Antibodies. | | |

Plasmatic levels of interleukin (IL)-6, IL-4, IL-17, IL-22, IL-23, monocyte chemotactic protein (MCP-1), Tumor necrosis factor alpha (TNFα), soluble TNF receptor II (sTNFRII) and vascular endothelial growth factor (VEGF) at baseline and 6 months after anti-TNFa treatment was quantified using cytofluorometry-based ELISA technique in accordance with manufacturer's instructions using FlowCytomix kit (eBioscience, USA). Results were calculated using the FlowCytometry Pro Software.

### Isolation of microRNAs from serum

Total RNA, including the miRNA fraction, was extracted from serum by using the QIAzol miRNeasy kit (Qiagen) with some modifications. 200 µl of serum were thawed on ice and lysed in 1000 µl QIAzol Lysis Reagent. Samples in QIAzol were incubated at room temperature for 5 min to inactivate RNases. To adjust for variations in RNA extraction and/or co-purification of inhibitors, 5 fmol of spike-in non-human synthetic miRNA (C. elegans miR-39 miRNA mimic) were added to the samples after the initial denaturation. C.elegans synthetic miRNA sequence used as spike-in controls was: Cel-miR-39 (5'-UCACCGGGUGUAAAUCAGCUUG-3'). The remaining extraction protocol was performed according to the manufacturer's instruction. Total RNA was eluted in 14 µl of RNase-free water and stored at -80°C.

### MicroRNA expression profiling

To identify the changes occurred in the expression levels of miRNAs in serum from patients treated with anti-TNFα drugs, Human Serum & Plasma miRNA PCR Array (Qiagen) was performed. This Array profiles the expression of 84 miRNAs detectable and differentially expressed in serum, plasma, and other bodily fluids. That miRNAs have been carefully selected based on published results that suggest a correlation with serum expression levels and specific diseases. A pool with 2 µl from RNA purified of 10 patients before treatment, and other pool with 2 µl from RNA purified of the same 10 patients after treatment was performed.

The miRNAs were reverse transcribed using the miScript II RT Kit (Qiagen). Raw data were analyzed with the data analysis software for miScript miRNA PCR Arrays available at http://pcrdataanalysis.sabiosciences.com/mirna. The expression levels of miRNAs were normalized to the mean of spiked- in miRNA Cel-miR-39 and was calculated using the 2-ΔΔCt method.

### Quantitative real-time PCR

A fixed volume of 3 µl of RNA solution from the 14 µl-eluate from RNA isolation of 200 µl serum sample was used as input into the reverse transcription. Input RNA was reverse transcribed using the TaqMan miRNA Reverse Transcription kit and miRNA-specific stem-loop primers (Applied BioSystems).The 15µl RT reaction was comprised of 3,15µl of H₂O, 1,5 µl of 10X Reverse-Transcription Buffer, 0,2µl of RNase-Inhibitor (20 units/µl), 0,15µl of 100mM dNTPs with dTTP, 1 µl of Multiscribe Reverse-Transcriptase, 1,5 µl of RT primers (4 miRNA RT primers/RT reaction), and 3 µl of input RNA. The reaction was conducted in a GeneAmp PCR System 9700 (Applied BioSystems) at 16°C for 30 min, 42°C for 30 min and 85°C for 5 min.

2,5 µl of RT product diluted (RT product with 10 µl of water) was combined with 5µl of Taqman 2x Universal PCR Master Mix, No AmpErase UNG and 2,5 µl of pool of 4 0,2X diluted Taqman miRNA Assay, to generate a Pre-Amplification Reaction of 10 µl (each Taqman miRNA Assay included in the pool remain at 0,05X in the Pre-Amplification reaction). The reaction was conducted in a GeneAmp PCR System 9700 (Applied BioSystems) at 95°C for 10 min, and 20 cycles of 95°C for 15 seconds and 60°C for 4 min.

Pre-Amplification product was diluted with 60 µl of water, and 4 µl were combined with 5 µl of Taqman 2x Universal PCR Master Mix, No AmpErase UNG, 0,5 µl of specific 20X Taqman miRNA Assay and 0,5 µl of water to generate a PCR of 10 µl of total volume. Real-time PCR was carried out on a Roche LightCycler 480 at 95°C for 10 min, followed by 40 cycles of 95°C for 15 s and 60°C for 1 min. Data were normalized to the mean of spiked-in miRNA Cel-miR-39 and the expression levels of miRNAs were calculated using the 2-ΔΔCt method.

### Statistical analysis

All data were expressed as mean ± SD. Statistical analyses were performed with SSPS 17.0 (SPSS Inc., Chicago, IL, USA). Following normality and equality of variance tests, clinical characteristics were compared using paired Student's t test or alternatively by a non-parametric test (Mann-Whitney rank sum test). Paired samples within the same subjects were compared by Wilcoxon signed-rank test. Correlations were assessed by Spearman's rank correlation. Differences were considered significant at p<0.05.

Receiver operating characteristic (ROC) curve analyses, plotting the true positive rate (sensitivity) vs. the false positive rate (1-specificity) at various threshold settings were performed for plasma miRNAs, and the areas under curve (AUCs) were calculated with SPSS. ROC analysis for miRNA-combined, arithmetic mean of level expression was calculated by two miRNA selected with highest efficiency values. P-values <0.05 were considered statistically significant.

### Results

### Clinical response to anti-TNF therapy

Within the cohort, 83 patients were female (87%) and 12 male (13%) with median disease duration of 9 (4-14) years. At the starting of the anti-TNF therapy all subjects showed high disease activity, reflected by a mean DAS28 of 5.67 (5.29-6.11) despite a median number of 2.35 DMARDS agents used concomitantly (range 1-3). All patients took non anti-inflammatory drugs daily and 65% of them received less than 10 mg/day prednisone. Methotrexate alone or in combination was administered in 81% of subjects.

According to DAS28 response criteria, 89.5% of patients were responders to anti-TNFa treatment. At 6 months of therapy most of the clinical parameters evaluated (including TJC, SJC, SDAI, and HAQ) improved significantly. All three biological agents had a favourable influence on the evolution of those parameters. Various autoimmune and serological parameters (such as RF, PCR, ESR, IL6, IL17, and TNFa) were further significantly reduced when patients were classified in responder's vs non-responders (Table II).

**Table II. Changes operated on clinical and laboratory parameters after anti-TNFa treatment in responders and non-responders AR patients**

| | **Responders (N=85)** | | | **Non-Responders (N=10)** | | |
|---|---|---|---|---|---|---|
| | **Before anti- TNF treatment** | **After anti-TNF treatment** | ***P*** | **Before anti- TNF treatment** | **After anti-TN F treatment** | ***P*** |
| ***Clinical Assesments*** | | | | | | |
| **TJC** | **15.9±4.8** | **5.1±2.2** | **0.000** | **15.6±5.1** | **8.5±2.9** | **0.005** |
| **SJC** | **11.7±3.9** | **2.9±1.8** | **0.000** | **11.6±4.9** | **5.1±2.7** | **0.005** |
| **DAS28** | **5.8±0.6** | **3.3±0.7** | **0.000** | **5.5±0.7** | **4.3±0.6** | **0.005** |
| **SDAI** | **36.3±11.4** | **2.9±6.8** | **0.000** | **39.2±11.8** | **5.7±12.1** | **0.005** |
| **HAQ** | **2.1±0.3** | **1±0.4** | **0.000** | **2.1±0.3** | **1±0.3** | **0.008** |

| ***Serological Assesments*** | | | | | | |
|---|---|---|---|---|---|---|
| **ESR (mm/h)** | **56.4±17.2** | **27.9± 18.5** | **0.000** | 51.4±11.9 | 37.6±19.3 | ns |
| **CRP (mg/L)** | **3.9±2.1** | **1.5±1.2** | **0.000** | 2.2±0.8 | 2.5±0.9 | ns |
| **RF (U/L)** | **155.2±288.3** | **85.4±241** | **0.000** | **61.2±84.3** | **20.8±39.9** | **0.027** |
| **IL-6 (pg/mL)** | **9.4±46.7** | **1.4±6.8** | **0.000** | 2.1±5.6 | 0 | ns |
| **TNF(pg/mL)** | **14.9±28.8** | **5.7±6.1** | **0.038** | 4.1±4.5 | 4.7±5.1 | ns |
| **sTNFRII (pg/mL)** | 1.5±0.9 | 1.5±0.7 | ns | 1.3±0.4 | 1.5±0.7 | ns |
| **MCP1 (pg/mL)** | 1174.8±1615.4 | 1216.4±1655.4 | ns | 740±504.6 | 678.6±221.8 | ns |
| **VEGF (pg/mL)** | 1107.7±1360.9 | 830.3±570.1 | ns | 796.7±462.7 | 1077.9±711.6 | ns |
| **IL23 (pg/mL)** | 115±235.5 | 76.8±132.9 | ns | 41.2±30.5 | 223.5±424.8 | ns |
| **IL22 (pg/mL)** | 46.3± 112 | 17.2± 29.2 | ns | 8.4±8.1 | 104.9±193.3 | ns |
| **IL17 (pg/mL)** | **4.8±8.5** | **2.1±2.1** | **0.024** | 11.9±19.1 | 1.4±1.4 | ns |
| **IL4 (pg/mL)** | 22.8±17.6 | 14.9±16.2 | ns | 4.2±6 | 12.8±18.2 | ns |

| | | | | | | |
|---|---|---|---|---|---|---|
| *T1*= *baseline, T2: at 6 months, TJC*=Tender Joint Count, *SJ*=Swolen Joint Count, DAS28=*Disease Activity Score, SDAI= Simplified Disease Activity Index,* HAQ=*Health Assessment Questionnaire,* ESR= Erythrocyte sedimentation rate, CRP= C-reactive protein . RF=Reumatoid Factor, IL6: Interleukina 6; TNF= Tumor Necrosis Factor; TNF-R: Receptor del TNF alfa; MCP-1: Proteína quimiotáctica de monocitos; VEGF: Factor de crecimiento endotelial vascular, IL23= Interleukina 23, IL22= Interleukina 22; IL17: Interleukina 17;IL$= Interleukina 4. | | | | | | |

### Differentially expressed miRNAs in the serum of RA patients before and after anti-TNF therapy

To evaluate the expression of serum miRNA before and after anti-TNF therapy, we profiled miRNA spectra from pools of 10 RA serum samples before treatment and 10 RA serum samples after treatment. We identified 84 miRNAs during this analysis process. In this profile, the expression levels of 75 miRNAS were found increased, while 9 miRNAs decreased after anti-TNF treatment (Figure 1A).

The detailed analysis of the miRNAs found increased in response to treatment with anti-TNF treatment, by using the Ingenuity Pathway Analysis (IPA, which includes various available databases to identify potential targets of these miRNAs - miRWalk, TargetScan, Miranda, miRTarBase and peer-reviewed microRNA original research articles as the content base for the microRNA Target Filter-, showed that a large number of them had target mRNAs involved in Immune and inflammatory response, cardiovascular system development and function or connective tissue and Musculoskeletal system. In the same way, those miRNAs were found mainly associated with inflammatory, cardiovascular, and connective tissue & musculoskeletal disorders (Figure 1B and 1C).

To validate the PCR Array data, five microRNAs differentially expressed, showing at least 2-fold change between the two conditions, were selected (hsa-miR-125b, hsa-miR-23a-3p, hsa-miR-21-5p, hsa-miR-126-3p and hsa-miR-146a-5p). A second group of five microRNAs under 2-fold change but involved in processes such us inflammation, cardiovascular and autoimmune diseases, and rheumatoid arthritis were also selected (hsa-let-7a-5p, hsa-miR-16-5p, hsa-miR-124a-3p, hsa-miR-155-5p, has-miR-223). Six of the ten miRNAs could clearly distinguish RA serum samples after anti-TNF therapy with high confidence (p<0.05): hsa-miR-125b, hsa-miR-126-3p, hsa-miR146a-5p, hsa-miR-16-5p, hsa-miR-23-3p, and hsa-miR-223) (Figure 2A).

These changed were found even more relevant when patients were divided in responders and non-responders, so that while miRNA expression levels strongly increased in responders, they did not change in non-responders patients after anti-TNF treatment (Figure 2B to 2G). Moreover, in responders, a parallel change was observed in the expression levels of autoimmune parameters such as the RF and of some cytokines such as TNFα, IL-6 or IL-17, which were found significantly reduced after anti-TNFa treatment (Table II).

It is widely accepted that miRNAs can influence gene expression by causing translational repression or mRNA degradation. This dysregulation can alter several downstream pathways and manifest effects. Therefore, by using the IPA analysis, gene targets predictions for the six validated miRNAs were performed in our study. Ingenuity Pathways uncovered the main enriched biological pathways (including Leukocyte activation and interaction, cytokine & chemokine signalling, bone remodelling, oxidative stress, and VEGF signalling) (Figure 3A), as well as the main biofunctions and disorders on which that miRNAs are involved (such as Immunological and inflammatory response and disease, cardiovascular, haematological, and connective tissue and musculoskeletal development and disorders) (Figure 3B).

### Serum miRNAs miR-223 and miR-223 as predictors of therapy response and biomarkers of response to therapy in RA patients.

As a general feature, we found that the better was the status of the patient before the treatment (in terms of clinical and serological parameters) the less changes in DAS and on the levels of miRNAs found after the anti-TNF therapy. In particular, elevated levels of miRNAs before starting the anti-TNF therapy were indicative of no response (Figure 4A).

This data is further supported by our ROC analysis, which showed that miR-23 and miR-223 levels at T1, with a cut-off value of 6.9 and 11.2 (relative expression at T1) were respectively predictors of non response to anti-TNF treatment (Figure 4B-C) with a sensitivity of 62.5% and 57.1%, and a specificity of 86.4% and 90.2% respectively.

The analysis of changes in the relative expression of miRNAs after anti-TNF-treatment further showed a down-regulation instead of up-regulation in RA patients non-responders, while in responders, a significant increase in three of the miRNAs validated was demonstrated (Figure 5A).

To evaluate their relevance as biomarkers of response to anti-TNF therapy, we conducted a ROC analysis of those miRNAs. Such ROC analysis showed the highest AUC for miR-23 and miR-223. Changed relative expression between T1 and T2 for miR-23, with a cutoff value of 0.83, demonstrated a sensitivity of 62.5% and a specificity of 77.6%. At a cutoff value of 3.03 for miR-223, the values were 57.1 % and 84.3 % respectively (Figure 5 A-C).

To improve accuracy of the analysis, we performed the combination of ROC curve analyses of miR-23, and miR-223.

The ratio of combination for these miRNAs at T1 demonstrated an increase in both the sensitivity (62.5%) and specificity (91.5%) in relation to those given by each miRNA alone (Figure 4B-C).

The ratio of combination for the change of these miRNAs (T1-T2) also yielded the highest AUC value of 0.88 and at the optimal cutoff value of 1.5, the sensitivity and specificity were 62.5% and 84.7 %, (Figure 5B-C).

Taken together, these results suggest that plasma miR-23 and miR-223 can act as both, predictors of therapy response, and biomarkers of response to anti-TNF therapy with high specificity.

### Regulation network of differentially expressed serum miRNAs in the inflammatory pathways and processes of Rheumatoid arthritis

The variation in serum miRNA expression levels before and after anti-TNF treatment suggested an association between miRNAs and key proteins underlying mechanisms related to both the physiopathology of RA and the response to the administered therapy. To better understand the possible biological pathways on which serum miRNAs might be involved, we constructed a miRNA-mRNA network to search the miRNA target genes that might function in the RA process. Ingenuity Pathways allowed us to uncover specific networks showing the interrelations among targets of the validated miRNAs directly associated to RA disease, including: i) altered T and B cell signalling; ii) role of macrophages, fibroblasts and endothelial cells in RA; iii) role of osteoblasts, osteoclasts and chondrocytes in RA; and iv) role of IL-17A in RA (Figures 6 and 7). Interestingly, a number of both miRNA and mRNA targets uncovered in the network, were found complementary altered after anti-TNF treatment in our patient's cohort.

### Changes in serum miRNAs correlate with changes in clinical variables in RA patients

To assess the possibility of serum miRNAs as biomarkers of RA and of response to therapy, we investigated the correlation of validated miRNAs with clinical and inflammatory variables. The changes observed in three miRNAs (miR-145a, miR-223 and miR-16) significantly correlated with the changes observed in clinical parameters (i.e.DAS28), and five of them at least with changes in inflammatory parameters such as CRP or ESR (miR-146a, miR-223, miR-16, miR-126 and miR-23) (Figure 8). A direct and significant relationship was also demonstrated among all that miRNAs (data not shown). In parallel, as above described, IPA analysis showed specific networks demonstrating interrelations among their targets directly associated to RA disease.

Micro-RNAs 23a and 223 as biomarkers for the prediction of specific responsiveness to treatment with anti-TNFalpha (infliximab, adalimumab and etanercept) agents in patients with rheumatoid arthritis.

The combination of expression levels of miRNAs 23a and 223 in the baseline period (T1) before treatment with anti-TNFa, are correlated negatively and significantly (p = 0.028) with the change in DAS28 (basic variable to assess the rheumatoid arthritis activity, hereinafter DAS) on patient after 6 months of treatment.

Thus, both miRNAs could be potential biomarkers predictors of response specific rate (based on decrease of DAS units) that would present a patient before starting treatment.

This fact could be a useful tool for the clinician in assessing whether to administer or not the drug to the patient in terms of efficiency. (Figure 1. Table contains the information of statistical significance).

In this way, if the degree of clinical response experienced by patients after treatment (DAST1-DAST2) versus units of change (udc) in three groups is stratified (less than 1.5 udc, between 1.5 and 3 udc, and more than 3 udc), show that there is a specific range of expression of the combination of both miRNAs in basal time (T1) that could predict each of the responses. Thus, high levels of these miRNAs in T1 (average = 7.8) would predict a poor response to treatment with a drop of less than 1.5 DAS. Average levels of these miRNAs in T1 (Average: 4.6) indicate that the patient could respond by presenting a drop between 1.5 and 3 in DAS units. The lowest levels of both miRNAs in T1 (Average: 3.4) could predict a better response to treatment reflected in a fall by more than 3 units in DAS (Figure 2).

Additionally the combination of these biomarkers with any clinical parameter would be potentially useful. Thus, the new parameter obtained of ratio between the combination of expression levels of both miRNAs in T1 with serum levels of C-reactive protein (CRP) in T1, allows discrimination of non-responders or responders, showing an area under ROC curve (AUC: 0.81) greater than that exhibited by the combined expression of the two miRNAs alone (AUC 0.76) (Figure 3).

Similarly, the ratio of the combined expression of miRNA 223 and 23a with CRP serum values prior to treatment with anti-TNFα, negatively and significantly (p = 0.004) correlated with the change DAS experienced by patients after 6 months of treatment. The generation of this new parameter could also be useful as a predictor biomarker of specific response rate (decrease based on DAS units) (Figure 4).

Thus, high levels of this ratio in T1 (average = 3.29) could predict a poor response to treatment with a drop of less than 1.5 DAS by stratified the response. Average levels of this ratio in T1 (Average: 1.57) indicate that the patient would respond by lowering between 1.5 and 3 units in DAS. A greater response to treatment reflected in a fall by more than 3 units in DAS could be predicted by the lowest levels in T1 ratio (average: 0.87) (Figure 5).

### Discussion

Micro-RNAs are emerging as potential targets for new therapeutic strategies of autoimmune disorders. In the present study, differentially expressed miRNAs in the serum of RA patients before and after anti-TNFα therapy, and their close relationship with the improvement of the clinical and serological markers of the disease, suggest that the expression levels of serum miRNAs have potential to serve as novel biomarkers for monitoring their therapy outcome.

Almost all of the RA patients showed complete clinical response to treatment with anti-TNF drugs, not only in clinical variables that reflect activity (swollen joints, painful pain scales, DAS28, etc.), but also in physical function, quality of life, fatigue, and sleep (HAQ). These results validate previous studies (Chen et al., 2013. Arthritis Care Res. Sep 10; Flouri et al., 2013. Semin. Arthritis Rheum. 2013).

The array allowed us to demonstrate that in response to treatment, most of the miRNAs evaluated (more than 90 %) were found increased. This could be related to the fact that miRNAs generally act as negative regulators of their target proteins, so that the increase in the levels of miRNAs could imply a reduction in levels of all the protein targets altered in their expression in RA patients before the biological therapy. In this regard, the functional classification allowed us to demonstrate that the majority of altered miRNAs had as potential target molecules / proteins / transcription factors involved in inflammation and autoimmunity processes, activation of T and B cells, musculoskeletal dysfunction or cardiovascular disease. Therefore, the increase in the levels of that miRNAs after anti-TNF treatment might be associated to a reduction in the inflammatory profile and the improvement of the overall disease status of those patients. In support for that hypothesis, we further found a significant reduction in the serum levels of inflammatory markers such as IL6, IL-17, VSG or PCR, as well as on the levels of markers of autoimmunity such as the RF.

A number of miRNAs were validated by RT-PCR in our cohort of patients (miR 146-a, miR-16, miR-23; miR-125b, miR223; miR126). Most of those miRNAs have been previously reported to act as relevant regulators of immune cells development, playing crucial roles in the inflammatory response, and acting as key players in the pathogenesis of various chronic and autoimmune disorders, including RA itself. (Nakamachi et al., 2009. Arthritis Rheum. 60(5), 1294-304; Nakasa et al., 2011. Arthritis Rheum. 2011 Jun; 63(6):1582-90; Wang et al., 2012. Transl. Res. 160(3), 198-206).

Thus, hsa-miR-146-a (whose main targets are IL6, IL17A, TLR4, IRAK-1, TRAF, or MCP-1) is involved in the differentiation and activation of T cells (Rusca et al., 2012. Mol. Cell Biol. 32(21), 4432-44), and modulates the expression and activity of cytokines in close association with the NFKB signaling pathway (Curtale et al., 2010. Blood 14; 115(2), 265-73). It has been also associated to the innate immune response (Luo et al., 2013. Arthritis Res. Ther. 9, 15(2), 210), and is induced in monocytes by LPS to control excessive immune activation (Liu et al., 2009. Zhongguo Wei Zhong Bing Ji Jiu Yi Xue. 22(9):540-542). As for disorders on which this miRNA has been concerned, it has been associated to the pathology SLE, RA, and psoriatic arthritis (Pauley et al., 2008. Arthritis Res. Ther. 10(4):R101; Chatzikyriakidou et al., 2010. Joint Bone Spine. 77(5):411-413; Chatzikyriakidou et al., 2010. Scand J Immunol. 2010 71(5), 382-5; Abou-Zeid et al., 2011. Genet Test Mol Biomarkers. 15(11), 807-812), as well as to atherosclerosis development (Raitoharju et al., 2011. Atherosclerosis 219(1), 211-217).

hsa-miR-125b (with IL6R, IL16, BCL 2, BMPR2, and NFATC4 as main targets) is involved in the differentiation of B cells (Gururajan et al., 2010. Int Immunol. 22(7), 583-92), in the regulation of TNF-alpha, and in inflammatory processes (Tili et al., 2007. J. Immunol. 15:179(8), 5082-9). It has been associated with Diabetes, Osteoarthritis and LES (Villeneuve et al., 2010. Diabetes 59(11):2904-15; Killock et al., 2013. Nat. Rev. Rheumatol. 9(4):198; Luo et al., 2013. Clin. Exp. Rheumatol. 31(2), 263-267).

hsa-miR-126 (whose main targets are VEGF, IL-21R, TGFBR2, MAPK8, TLR4 and JAK2), regulates the induction and function of CD4(+) Foxp3(+) regulatory T cells (Qin et al., 2013. J. Cell Mol. Med. 17(2), 252-64) and is involved in vascular inflammation (Wang et al., 2012. Braz. J. Med. Biol. Res. 45(12):1308-14). It has been described as potential biomarker in serum of SLE patients, iskemic stroke and coronary artery disease (Zhao et al., 2011. Arthritis Rheum. 63(5), 1376-1386; Long et al., 2013. BMC Neurol. 16, 13:178; Sun et al., 2012 Thromb. J. 28;10(1):16), and as a potential therapeutic tool in atherosclerosis (Schober et al., 2014 Nat Med. 2).

hsa-miR-223 (including targets such as TNF, IL-10, IL-17a, IL-6, JAK2, NFKB1, TLR4, among others) which was first described as a modulator of hematopoietic lineage differentiation, has also an emerging role in inflammatory and metabolic disorders, with a particular focus on muscle diseases, type II diabetes, atherosclerosis and vascular calcification (Taïbi et al., 2014. Biochim. Biophys. Acta. S0925-4439(14)00065-9). Candidate biomarker in lupus patients (Carlsen et al., 2013. Arthritis Rheum. 65(5):1324-34), it has been particularly associated with the onset of RA (Sebastiani et al., 2011. Clin. Exp. Rheumatol. 29(6):1058-9; Okuhara *et al.,* 2012. 22(3):446-57; Chatzikyriakidou et al., 2012. Autoimmun. Rev. 11(9): 636-41; Shibuya et al., 2012. Mod. Rheumatol. 23(4), 674-85).

hsa-miR-16 (having as main targets CD28, IL15, IL36B, IRAK2, FGF2, VEGFA, and IL-6) has been shown to be implicated in apoptosis induction (Cimmino et al., 2005. Proc. Natl. Aca. Sci. USA 102:13944-949*)* in cell-cycle regulation (Linsley et al., 2007. Mol. Cell Biol. 27, 2240-52) and angiogenesis (Karaa et al., 2009 RNA 15:249-54). Candidate biomarker in pathologies such as SLE (Wang et al., 2012. Transl. Res. 160(3), 198-206) osteorarthritis (Murata et al., 2010. Arthritis Res. Ther. 12(3):R86), and ankilosing spondilitys (Lai et al., 2013. Clin. Exp. Immunol. 173(1):47-57), among others, it has been demonstrated to be differentially expressed in serum and circulating leukocytes in RA patients, and directly associated to the susceptibility and the disease activity. (Chatzikyriakidou et al., 2012. Autoimmun. Rev. 11(9): 636-41; Filková et al., 2013. Ann. Rheum. Dis. 29).

Finally, hsa-miR-23a-3p (main targets: IL6R, IL11, CCL7, CXCL12, FGF2, PDGFA, BMPR2) has been involved in multiple processes, including the contribution to dyslipidemia in metabolic syndrome (Karolina et al., 2012. J. Clin. Endocrinol. Metab. 97(12):E2271-6), the regulation of TNFα-induced apoptosis in mesenchymal stem cells (Mao et al., 2013. Exp. Mol. Pathol. 19 pii:S0014-4800(13)00139-1), the control of mesenchymal lineage progression, (Zhang et al., 2012. J. Biol. Chem. 22, 287(26):21926-35) and the regulation of the osteoblasts differentiation program (Hassan et al., 2010. Proc. Natl. Acad. Sci. USA. 16, 107(46), 19879-84). Concomitantly, it has been associated to cardiac hypertrophy (Han et al., 2011. Curr. Opin. Cardiol. 26(3), 181-9; Wang et al., 2012. J. Biol. Chem. 2; 287(1), 589-99), myocardial infarction (Mao et al., 2013. Exp. Mol. Pathol. 19 pii: S0014-4800(13)00139-1) osteoporosis (Seeliger et al., 2014. J. Bone Miner. Res. 16. doi: 10.1002/jbmr.2175), and multiple sclerosis (Fenoglio et al., 2013. Mult. Scler. 19(14):1938-42).

Consistently with our results , a recent study has shown the association of two of the microRNAs found significantly increased in response to anti-TNFa therapy in our study (miR- 223, and miR -16) with disease activity in RA patients newly diagnosed (Filková et al., 2013. Ann. Rheum. Dis. 29). Furthermore, three trials in 2008 indicated the existence of high levels of some of those miRNAs (miR -16, miR -132, miR- 146a and miR-155) in leukocytes of arthritic patients (Pauley et al., 2008. Ann. N. Y. Acad. Sci. 1143, 226-239). A more recent study showed that circulating levels of miR146a and miR155 were significantly down-regulated in patients with RA in relation to healthy donors (Wang et al., 2012. Transl. Res. 160(3), 198-206).

In support for that previously reported data, correlation studies in our cohort demonstrated, firstly, the existence of a significant relationship among all the validated miRNAs. Moreover, all of them have putative targets directly associated to RA disease. Secondly, we found a negative correlation between the changes in the expression levels of almost all the validated miRNAs and the changes occurred in various clinical and inflammatory parameters. Furthermore, ROC analyses demonstrated that two of these six miRNAs (miR-23 and miR-223) can act in RA patients as both, predictors of therapy response, (indicating what patients would not benefit from anti-TNF treatment), and as biomarkers of response to anti-TNF therapy, so that their levels would indicate if the treatment has been effective.

Our data contrast with a recent study performed in patients with psoriasis treated with the TNF-inhibitor etanercept (Pivarcsi et al., 2013. Br. J. Dermatol. 169(3):563-70). In that cohort of patients etanercept significantly downregulated serum levels of miR-223 and miR-126 among others. In addition, those miRNAs were not related to disease severity in psoriasis. Thus, those results suggest a distinctive involvement of similar miRNAs in pathways affected by anti-TNFα therapy depending on the inflammatory disease concerned.

Altogether, our data suggest that differentially expressed miRNAs in the serum of RA patients before and after anti-TNFα therapy have potential to serve as novel biomarkers for monitoring the outcome of the therapy.

## Claims

1. The *in vitro* use of SEQ ID NO: 2 (miR-23a) as a biomarker for predicting response of a human subject suffering from rheumatoid arthritis to anti-TNFα therapy, comprising determining the expression level thereof.

2. The *in vitro* use according to claim 1, wherein the expression levels of SEQ ID NO: 2 (miR-23a) are used in combination with the expression levels of SEQ ID NO: 6 (miR-223) as biomarkers for predicting response of a human subject suffering from rheumatoid arthritis to anti-TNFα therapy.

3. The use according to claim 2, further comprising using, as biomarkers, the expression levels of one or more miRNAs as set forth by the following SEQ ID NOs:
(i) SEQ ID NO: 1 (miR-16);
(ii) SEQ ID NO: 3 (miR125b);
(iii) SEQ ID NO: 4 (miR-126a);
(iv) SEQ ID NO: 5 (miR-146a).

4. A method of predicting response of a human subject suffering from rheumatoid arthritis to anti-TNFa therapy, comprising using, as an indicator, expression levels of SEQ ID NO: 2 (miR-23a) and obtaining a result of the method by determining in samples obtained from said human subject the changed relative expression between T1 and T2 for SEQ ID NO:2; wherein T1 is understood as prior to subject initiating anti-TNFa therapy and T2 is understood as after the subject has initiated anti-TNFa treatment; and wherein the result is indicative for response if the expression in T2 has increased relative to the expression in T1.

5. A method of predicting response of a human subject suffering from rheumatoid arthritis, to anti-TNFα therapy comprising using, as an indicator, expression levels of SEQ ID NO: 2 (miR-23a) and obtaining a result of the method by determining the expression levels at T1 and comparing it with a cut-off value; wherein T1 is understood as prior to the subject initiating anti-TNFα treatment; and wherein the result is indicative for response if the expression in T1 has increased relative to the cut-off value.

6. The method of claim 4 wherein the method comprises using, as an indicator, the expression levels of SEQ ID NO: 2 (miR-23a) in combination with the expression levels of SEQ ID NO: 6 (miR-223), and obtaining a result of the method by determining the changed relative expression between T1 and T2 for SEQ ID NO:2 and SEQ ID NO: 6; wherein T1 is understood as prior to subject initiating anti-TNFα therapy and T2 is understood as after the subject has initiated anti-TNFα treatment; and wherein the result is indicative for response if the expression of both biomarkers in T2 has increased relative to the expression in T1.

7. A method of predicting response of a human subject suffering from rheumatoid arthritis, to anti-TNFα therapy comprising using, as an indicator, expression levels of SEQ ID NO: 2 (miR-23a) in combination with expression levels of SEQ ID NO: 6 (miR-223) and obtaining a result of the method by determining the expression levels at T1 and comparing it with a cutoff value, wherein T1 is understood as prior to the subject initiating anti-TNFα treatment, and wherein the result is indicative for response if the expression of both biomarkers in T1 has increased relative to the cutoff value.

8. The method according to any one of claims 4-7, wherein said result is obtainable by
(i) a gene profiling method, such as a microarray, and/or
(ii) a method comprising PCR, such as real time PCR; and/or
(iii) Northern Blot.

9. The method according to any of claims 4 to 7, wherein the result is obtainable by quantitative real-time PCR and expressed as -ΔΔCt.

10. The method according to any of claims 4 to 9, wherein, the method is performed *in vitro* using a plasma or blood sample originating from the human subject.

11. The method according to any of claims 4 to 10, wherein the anti-TNFa therapy comprises administration of infliximab, adalimumab, certolizumab, golimumab, etanercept, or combinations thereof.

12. A method for allocating a human subject suffering from rheumatoid arthritis in one of two groups, wherein group 1 comprises responders subjects identifiable by the method according to any one of claims 4 to 11, and wherein group 2 represents the remaining subjects.

13. A pharmaceutical composition comprising an anti-TNFα agent or TNF inhibitor, for use in a method of treatment of responder patients suffering from rheumatoid arthritis which are identified by the method of claim 12.

14. *In vitro* use of a kit comprising at least one oligonucleotide(s) capable of hybridizing with miRNAs set forth as SEQ ID NOs: 2 under stringent conditions in a method according to any one of claims 4 to 12.

15. The *in vitro* use according to claim 14, wherein the further comprises oligonucleotide(s) capable of hybridizing with miRNAs set forth as SEQ ID 6 and optionally NOs: 1, 3, 4, and/or 5, under stringent conditions.

## Patentansprüche

1. *In-vitro*-Verwendung von SEQ ID NO: 2 (miR-23a) als Biomarker zur Vorhersage der Reaktion eines an rheumatoider Arthritis leidenden menschlichen Subjekts auf eine Anti-TNFα-Therapie, umfassend das Bestimmen des Expressionsniveaus davon.

2. *In-vitro*-Verwendung nach Anspruch 1, wobei die Expressionsniveaus von SEQ ID NO: 2 (miR-23a) in Kombination mit den Expressionsniveaus von SEQ ID NO: 6 (miR-223) als Biomarker zur Vorhersage der Reaktion eines an rheumatoider Arthritis leidenden menschlichen Subjekts auf eine Anti-TNFα-Therapie verwendet werden.

3. Verwendung nach Anspruch 2, ferner umfassend die Verwendung des Expressionsniveaus eines oder mehreren miRNAs, als Biomarker, wie durch die folgenden SEQ ID NO dargelegt:
(i) SEQ ID NO: 1 (miR-16);
(ii) SEQ ID NO: 3 (miR-125b);
(iii) SEQ ID NO: 4 (miR-126a);
(iv) SEQ ID NO: 5 (miR-146a).

4. Verfahren zur Vorhersage der Reaktion eines an rheumatoider Arthritis leidenden menschlichen Subjekts auf eine Anti-TNFα-Therapie, umfassend die Verwendung der Expressionsniveaus von SEQ ID NO: 2 (miR-23a), als Indikator, und die Erhaltung eines Ergebnisses des Verfahrens durch Bestimmen der veränderten relativen Expression zwischen T1 und T2 für SEQ ID NO:2 in von dem menschlichen Subjekt erhaltenen Proben; wobei T1 als vor dem Einleiten einer Anti-TNFα-Therapie bei einem Subjekt verstanden wird und T2 als nachdem eine Anti-TNFα-Behandlung bei einem Subjekt eingeleitet worden ist, verstanden wird; und wobei das Ergebnis auf eine Reaktion hinweist, wenn sich die Expression in T2 relativ zu der Expression in T1 erhöht hat.

5. Verfahren zur Vorhersage der Reaktion eines an rheumatoider Arthritis leidenden menschlichen Subjekts auf eine Anti-TNFα-Therapie, umfassend die Verwendung der Expressionsniveaus von SEQ ID NO: 2 (miR-23a), als Indikator, und die Erhaltung eines Ergebnisses des Verfahrens durch Bestimmen der Expressionsniveaus bei T1 und Vergleichen mit einem Ausschlusswert; wobei T1 als vor dem Einleiten einer Anti-TNFα-Behandlung bei einem Subjekt verstanden wird; und wobei das Ergebnis auf eine Reaktion hinweist, wenn sich die Expression in T1 relativ zu dem Ausschlusswert erhöht hat.

6. Verfahren nach Anspruch 4, wobei das Verfahren die Verwendung der Expressionsniveaus von SEQ ID NO: 2 (miR-23a) in Kombination mit den Expressionsniveaus von SEQ ID NO: 6 (miR-223), als Indikator, umfasst, und die Erhaltung eines Ergebnisses des Verfahrens durch Bestimmen der veränderten relativen Expression zwischen T1 und T2 für SEQ ID NO:2 und SEQ ID NO: 6; wobei T1 als vor dem Einleiten einer Anti-TNFα-Therapie bei einem Subjekt verstanden wird und T2 als nachdem eine Anti-TNFα-Behandlung bei einem Subjekt eingeleitet worden ist, verstanden wird; und wobei das Ergebnis auf eine Reaktion hinweist, wenn sich die Expression beider Biomarker in T2 relativ zu der Expression in T1 erhöht hat.

7. Verfahren zur Vorhersage der Reaktion eines an rheumatoider Arthritis leidenden menschlichen Subjekts auf eine Anti-TNFα-Therapie, umfassend die Verwendung der Expressionsniveaus von SEQ ID NO: 2 (miR-23a) in Kombination mit den Expressionsniveaus von SEQ ID NO: 6 (miR-223), als Indikator, und die Erhaltung eines Ergebnisses des Verfahrens durch Bestimmen der Expressionsniveaus bei T1 und Vergleichen mit einem Ausschlusswert, wobei T1 als vor dem Einleiten einer Anti-TNFα-Behandlung bei einem Subjekt verstanden wird, und wobei das Ergebnis auf eine Reaktion hinweist, wenn sich die Expression beider Biomarker in T1 relativ zu dem Ausschlusswert erhöht hat.

8. Verfahren nach einem der Ansprüche 4-7, wobei das Ergebnis erhältlich ist durch
(i) ein Genprofilierungsverfahren, wie ein Mikroarray, und/oder
(ii) ein Verfahren, umfassend PCR, wie Echtzeit-PCR; und/oder
(iii) Northern Blot.

9. Verfahren nach einem der Ansprüche 4 bis 7, wobei das Ergebnis durch quantitative Echtzeit-PCR erhältlich ist und als -ΔΔCt ausgedrückt wird.

10. Verfahren nach einem der Ansprüche 4 bis 9, wobei das Verfahren *in vitro* durchgeführt wird, unter Verwendung einer von dem menschlichen Subjekt stammenden Plasma- oder Blutprobe.

11. Verfahren nach einem der Ansprüche 4 bis 10, wobei die Anti-TNFα-Therapie die Verabreichung von Infliximab, Adalimumab, Certolizumab, Golimumab, Etanercept, oder Kombinationen davon umfasst.

12. Verfahren zum Zuweisen eines an rheumatoider Arthritis leidenden menschlichen Subjekts in eine von zwei Gruppen, wobei Gruppe 1 Responder-Subjekte umfasst, die durch das Verfahren nach einem der Ansprüche 4 bis 11 identifizierbar sind, und wobei Gruppe 2 die restlichen Subjekte darstellt.

13. Pharmazeutische Zusammensetzung, umfassend ein Anti-TNFα-Mittel oder TNF-Hemmer, zur Verwendung in einem Verfahren zur Behandlung von an rheumatoider Arthritis leidenden Responder-Patienten, die durch das Verfahren nach Anspruch 12 identifiziert werden.

14. *In-vitro*-Verwendung eines Kits, umfassend mindestens ein Oligonukleotid (mehrere Oligonukleotide), das/die mit als SEQ ID NO: 2 dargelegten miRNAs, unter stringenten Bedingungen in einem Verfahren nach einem der Ansprüche 4 bis 12, hybridisieren kann/können.

15. *In-vitro*-Verwendung nach Anspruch 14, ferner umfassend ein Oligonukleotid (mehrere Oligonukleotide), das/die mit als SEQ ID NO: 6 und gegebenenfalls SEQ ID NO: 1,3, 4 und/oder 5 dargelegten miRNAs, unter stringenten Bedingungen hybridisieren kann/können.

## Revendications

1. Utilisation *in vitro* de la SEQ ID NO : 2 (miR-23a) en tant que biomarqueur pour prédire la réponse d'un sujet humain souffrant de polyarthrite rhumatoïde à une thérapie anti-TNFα, comprenant la détermination de son taux d'expression.

2. Utilisation *in vitro* selon la revendication 1, dans laquelle les taux d'expression de la SEQ ID NO : 2 (miR-23a) sont utilisés en combinaison avec les taux d'expression de la SEQ ID NO : 6 (miR-223) en tant que biomarqueurs pour prédire la réponse d'un sujet humain souffrant de polyarthrite rhumatoïde à une thérapie anti-TNFα.

3. Utilisation selon la revendication 2, comprenant en outre l'utilisation, en tant que biomarqueurs, des taux d'expression d'un ou de plusieurs miARN tels que définis par les SEQ ID NO suivantes :
(i) SEQ ID NO : 1 (miR-16) ;
(ii) SEQ ID NO : 3 (miR125b) ;
(iii) SEQ ID NO : 4 (miR-126a) ;
(iv) SEQ ID NO : 5 (miR-146a).

4. Méthode de prédiction de la réponse d'un sujet humain souffrant de polyarthrite rhumatoïde à une thérapie anti-TNFα, comprenant l'utilisation, en tant qu'indicateur, de taux d'expression de la SEQ ID NO : 2 (miR-23a) et l'obtention d'un résultat de la méthode en déterminant, dans des échantillons obtenus auprès dudit sujet humain, l'expression relative modifiée entre T1 et T2 pour la SEQ ID NO: 2 ; dans laquelle il est entendu queT1 est antérieur à l'initiation de la thérapie anti-TNFα chez le sujet et il est entendu queT2 est postérieur à l'initiation de la thérapie anti-TNFα chez le sujet ; et dans laquelle le résultat est indicatif de la réponse si l'expression dans T2 a augmenté par rapport à l'expression dans T1.

5. Méthode de prédiction de la réponse d'un sujet humain souffrant de polyarthrite rhumatoïde à une thérapie anti-TNFα comprenant l'utilisation, en tant qu'indicateur, de taux d'expression de la SEQ ID NQ : 2 (miR-23a) et l'obtention d'un résultat de la méthode en déterminant les taux d'expression à T1 et en les comparant avec une valeur limite; dans laquelle il est entendu queT1 est antérieur à l'initiation de la thérapie anti-TNFα chez le sujet ; et dans laquelle le résultat est indicatif de la réponse si l'expression dans T1 a augmenté par rapport à la valeur limite.

6. Méthode selon la revendication 4, la méthode comprenant l'utilisation, en tant qu'indicateur, des taux d'expression de la SEQ ID NO : 2 (miR-23a) en combinaison avec les taux d'expression de la SEQ ID NO : 6 (miR-223), et l'obtention d'un résultat de la méthode en déterminant l'expression relative modifiée entre T1 et T2 pour les SEQ ID NO : 2 et SEQ ID NO : 6 ; dans laquelle il est entendu queT1 est antérieur à l'initiation de la thérapie anti-TNFα chez le sujet et il est entendu queT2 est postérieur à l'initiation de la thérapie anti-TNFα chez le sujet ; et dans laquelle le résultat est indicatif de la réponse si l'expression des deux biomarqueurs dans T2 a augmenté par rapport à l'expression dans T1.

7. Méthode de prédiction de la réponse d'un sujet humain souffrant de polyarthrite rhumatoïde à une thérapie anti-TNFα, comprenant l'utilisation, en tant qu'indicateur, de taux d'expression de la SEQ ID NO : 2 (miR-23a) en combinaison avec les taux d'expression de la SEQ ID NO : 6 (miR -223) et l'obtention d'un résultat de la méthode en déterminant les taux d'expression à T1 et en les comparant avec une valeur limite ; dans laquelle il est entendu queT1 est antérieur à l'initiation de la thérapie anti-TNFα chez le sujet ; et dans laquelle le résultat est indicatif de la réponse si l'expression dans T1 a augmenté par rapport à la valeur limite.

8. Méthode selon l'une quelconque des revendications 4 à 7, dans laquelle ledit résultat peut être obtenu par
(i) une méthode de profilage de gènes, telle qu'un microréseau, et/ou
(ii) une méthode comprenant une PCR, telle qu'une PCR en temps réel ; et/ou
(iii) un transfert de Northern.

9. Méthode selon l'une quelconque des revendications 4 à 7, dans laquelle le résultat peut être obtenu par PCR quantitative en temps réel et exprimé par -ΔΔCt.

10. Méthode selon l'une quelconque des revendications 4 à 9, la méthode étant réalisée *in vitro en* utilisant un plasma ou un échantillon de sang provenant du sujet humain.

11. Méthode selon l'une quelconque des revendications 4 à 10, dans laquelle la thérapie anti-TNFα comprend l'administration d'infliximab, d'adalimumab, de certolizumab, de golimumab, d'étanercept ou de combinaisons de ceux-ci.

12. Méthode de répartition d'un sujet humain souffrant de polyarthrite rhumatoïde dans l'un des deux groupes, dans laquelle le groupe 1 comprend les sujets répondeurs identifiables par la méthode selon l'une quelconque des revendications 4 à 11, et dans laquelle le groupe 2 représente les sujets restants.

13. Composition pharmaceutique comprenant un agent anti-TNFα ou un inhibiteur du TNF, destinée à être utilisée dans une méthode de traitement de patients répondeurs souffrant de polyarthrite rhumatoïde qui sont identifiés par la méthode de la revendication 12.

14. Utilisation *in vitro* d'un kit comprenant au moins un oligonucléotide capable de s'hybrider avec les miARN définis en tant que SEQ ID NO : 2 dans des conditions strictes dans une méthode selon l'une quelconque des revendications 4 à 12.

15. Utilisation *in vitro* selon la revendication 14, qui comprend en outre un ou plusieurs oligonucléotides capables de s'hybrider avec les miARN définis en tant que SEQ ID NO: 6 et facultativement NO: 1,3, 4 et/ou 5, dans des conditions strictes.
